# EUROPEAN PATENT SPECIFICATION

(11) **EP 3 116 569 B1**
(45) Date of publication and mention of the grant of the patent: **28.02.2018**
(21) Application number: 15715459.2
(22) Date of filing: 10.03.2015
(51) Int. Cl.: A61M 15/00

(54) **ACTIVE DRY POWDER INHALER**
INHALATOR FÜR AKTIVES TROCKENPULVER
INHALATEUR ACTIF DE POUDRE SÈCHE

(30) Priority: 10.03.2014 US 201461950256 P
(43) Date of publication of application: 18.01.2017
(73) Proprietor: Inspiro Medical Ltd., 7403650 Nes Ziona (IL)
(72) Inventor: KAUFMANN, Nimrod, 7171890 ModiIn (IL)
(74) Representative: Luppi, Emanuele
(86) International application number: PCT/IL2015/050248
(87) International publication number: WO 2015/136529

(56) References cited:
- EP-A2- 0 796 628
- EP-A2- 1 321 160
- WO-A1-2011/133740
- DE-A1- 19 502 725
- DE-B3-102005 043 449
- DE-U1-202011 104 129
- GB-A- 1 436 028
- US-A- 2 946 332
- US-A- 5 989 217
- US-A1- 2011 220 106
- US-A1- 2013 267 864
- US-B1- 6 398 074

## Description

### FIELD AND BACKGROUND OF THE INVENTION

The present invention, in some embodiments thereof, relates to the field of dry powder delivery devices, and more particularly, to methods and systems for controlled delivery of dry powder.

Inhalation of therapeutic aerosols or dry powder medicaments are an effective method of drug delivery, frequently applied to treat respiratory disease. Three leading types of inhalers are commonly used today: metered dose inhalers, dry powder inhalers, and nebulizers.

In a Metered Dose Inhaler (MDI), a solution is stored in a pressurized canister that contains a propellant. The canister is mounted to a hand-operated actuator having a mouthpiece and optionally a chamber. The user exhales, and then places the mouthpiece or the chamber in the mouth, actuating the device to inhale a fixed dose of medication in aerosol form.

A Dry Powder Inhaler (DPI) inhaler delivers a medication in the form of a dry powder to the lungs. A measured quantity of medication is manually loaded into the inhaler prior to use, or pre-loaded inside the inhaler. The user inserts the inhaler mouthpiece into the mouth, takes a deep inhalation, and holds the breath for few seconds to allow the inhaled particles to settle onto the lung airways. Most DPI devices relay on the patient inhalation flow rate to transfer the powder from the device (as a gas suspension of solid particles), and to deagglomerate the powder into aerosol particles small enough to reach the lungs. An insufficient inhalation flow may result in an inadequate dose delivery and incomplete powder deagglomeration.

Nebulizers deliver drug in the form of aerosol. Compressed air is blasted through or near a liquid medicine to aerosolize it. The aerosol is then inhaled by the patient using a mouthpiece or a face mask. Some nebulizers use vibrating membranes instead of a compressor to produce the aerosol. Nebulizers are designed for a prolonged respiratory cycle drug delivery. While inhaling the aerosol medicaments, the user breathes in a normal rate for several minutes until the dose transfer is completed.

Dry powder inhalers as known in the art are exemplified in the following publications:
- A U.S. Patent Application Publication No. 2004/0089299, to Bonney et al.*,* entitled "Inhaler".
- A U.S. Patent No. 6,012,454 to Hodson et al.*,* and entitled "Dry Powder Inhalation Device".
- A U.S. Patent Application Publication No. 2007/272763 to Dunne et al.*,* and entitled "Dispensing Device, Storage Device and Method for Dispensing Powder".
- A U.S. Patent Application Publication No. 2005/263151 to Hochrainer et al.*,* and entitled "Powder Inhaler Having a Nozzle with a Plurality of Channels".
- A U.S. Patent Application Publication No. 2001/029948 to Ingle et al.*,* and entitled "Systems and Methods for Extracting Powders from Receptacles".
- A U.S. Patent Application Publication No. 2005/087189 to Crockford et al.*,* and entitled "Drug Delivery Apparatus".
- A U.S. Patent Application Publication No. 2008/035143 to Sievers et al.*,* and entitled "Human-Powered Dry Powder Inhaler and Dry Powder Inhaler Compositions".
- An International Patent Application Publication No. WO2002/005879 to Brand et al.*,* and entitled "Medicament Dispenser".
- An International Patent Application Publication No. WO2011/077414 to Kaufmann et al.*,* and entitled "Dry Powder Delivery Device and Method".

Additional background art includes: U.S. Patent No. 7,819,116; United States Patent No. 7,520,278; U.S. Patent No. 7,458,373; U.S. Patent No. 7,322,355; U.S. Patent No. 7,117,867; U.S. Patent Application Publication No. 2009/0095294; U.S. Patent Application Publication No. 2005/0056276; U.S. Patent Application Publication No. 2004/0079368; and U.S. Patent Application Publication No. 2003/0168057.

Also comprising background art are German Patent Publication No. 19502725 A1, relating to a pump dispenser unit for delivering a medium carried in a fluid from a holding zone; and U.S. Patent Application Publication No. 2011/0220106 A1, relating to a medication inhaler for concurrently delivering multiple doses of medications, including a medication delivery needle which penetrates the medication containers when the inhaler is actuated.

Further background art includes U.S. Patent 2,946,332, relating to an insufflator comprising a lance member applied to a nozzle and urged to its lowermost position so that the needle end pierces and passes completely through the opposite ends of the capsule.

### SUMMARY OF THE INVENTION

The invention is defined in the appended independent claims 1, 12 and 14. According to an aspect of the present invention, there is provided an inhaler for delivering dry powder medicament contained in a capsule as defined in claim 1, the inhaler comprising: a capsule chamber sized to fittingly hold the capsule within a capsule-holding volume; and at least one needle; wherein the needle moves along a path to introduce a portion of the needle across a first boundary location of the capsule-holding volume, and into an interior of the capsule-holding volume; and wherein the needle moves further along the path to move the portion of the needle from the interior to a second boundary location of the capsule-holding volume, the moving further along the path forces the portion of the needle against a wall of the capsule from within the interior to form a capsule outlet for the dry powder medicament. Afterwards, the portion of the needle moves past the second boundary location to exit the capsule-holding volume, and then the portion of the needle retracts back into the interior, away from the second boundary location, leaving an exit aperture open. According to some aspects, no more than 20% of the wall of the capsule displaced to form the capsule outlet is within the capsule interior after formation of the capsule outlet.

According to some embodiments of the invention, the shape and size of the capsule outlet is determined by the cross-section of the needle.

According to some embodiments of the invention, the needle comprises cross-sections of at least two different sizes.

According to some embodiments of the invention, the two different cross-sections occur on a respective at least two longitudinal sections, each having a different cross-section therealong.

According to some embodiments of the invention, a tip of the needle portion comprises a sharpened conical section.

According to some embodiments of the invention, the needle comprises a lumen having a gas inlet and a gas outlet; and the outlet moves into the capsule-holding volume when the needle moves along the path into the interior.

According to some embodiments of the invention, the inhaler comprises a compressed gas source and a delivery system for gas from the compressed gas source to the lumen of the needle.

According to some embodiments of the invention, the inhaler comprises at least two spatially separated gas outlets.

According to some embodiments of the invention, the inhaler comprises at least one valve for separately controlling gas delivery via each of the gas outlets.

According to some embodiments of the invention, the needle moves to position the gas outlet in at least two positions within the capsule-holding volume from which gas is delivered into the capsule-holding volume.

According to some embodiments of the invention, the inhaler further comprises a controller functionally connected with the gas delivery system for controlling delivery of compressed gas to the gas outlet.

According to some aspects, the inhaler further comprises a flow sensor functionally connected with the controller; the controller determining breathing parameters of a user of the inhaler according to flow rate measurements of the flow sensor; the controller controlling the delivery of the compressed gas into the capsule, and thereby controlling delivery of the medicament dry powder to the user of the inhaler, in accordance with the determined breathing parameters.

According to some aspects, the breathing parameters are selected from the list consisting of: inspiratory flow rate; inspiratory volume; inspiratory phase; expiratory flow rate; expiratory volume; expiratory phase; and breath pattern.

According to some aspects, the inhaler further comprises a feedback interface coupled with the controller, the controller providing breathing guidance to the user, via the feedback interface.

According to some aspects, the feedback interface operates to provide user breathing guidance, prior to the needle moving along the path.

According to some aspects, the inhaler can deliver more than a single dry powder medicament simultaneously.

According to some aspects, the capsule chamber fittingly receives a group of physically linked capsules, forming together a continuous chain of powder compartments.

According to an aspect of the present invention, there is provided a detachable module as defined in claim 12, the module fittable to an inhaler for delivering dry powder medicament contained in a capsule, the module comprising: a capsule chamber sized to fittingly hold the capsule within a capsule-holding volume; and at least one needle; wherein the needle moves along a path to introduce a portion of the needle across a first boundary location of the capsule-holding volume, and into an interior of the capsule-holding volume; and wherein the needle moves further along the path to move the portion of the needle from the interior to a second boundary location of the capsule-holding volume.

According to an aspect of some embodiments of the present invention, there is provided a kit comprising the module, and at least ten capsules containing dry powder medicament.

According to an aspect, there is provided an inhaler for delivering dry powder medicament contained in a capsule, the inhaler comprising: a capsule chamber sized to fittingly hold the capsule within a capsule-holding volume; and at least one needle comprising a gas lumen having at least one gas outlet which moves into the capsule-holding volume, and from which gas is introduced into the capsule-holding volume.

According to some aspects, the at least one gas outlet comprises at least two spatially separated gas outlets.

According to some aspects, the inhaler comprises at least one valve for separately controlling gas delivery via each of the at least two spatially separated gas outlets.

According to some aspects, the needle is movable within the capsule-holding volume so that the at least one gas outlet releases gas from at least two positions within the capsule-holding volume.

According to some aspects, the inhaler produces a plurality of gas flow regimes within the capsule from the at least one gas outlet by separately controlling the gas flow from each outlet.

According to some aspects, at least one of the plurality of flow regimes comprises a vortex which substantially fills the interior of the capsule with gas rotating in a first rotational direction.

According to some aspects, at least one of the plurality of flow regimes comprises gas flowing in a plurality of rotational directions.

According to some aspects, at least one of the plurality of flow regimes comprises a vortex which substantially fills the interior of the capsule with gas rotating in a second rotational direction.

According to an aspect, there is provided a detachable module fittable to an inhaler for delivering dry powder medicament contained in a capsule, the module comprising: a capsule chamber sized to fittingly hold the capsule within a capsule-holding volume; and at least one needle comprising a gas lumen having at least one gas outlet which moves into the capsule-holding volume, and from which gas is introduced into the capsule-holding volume.

According to an aspect, there is provided a kit comprising the module, and at least ten capsules containing dry powder medicament.

According to an aspect of the present invention, there is provided a method of preparing an inhaler capsule containing dry powder medicament with a capsule outlet for the medicament as defined in claim 14, the method comprising: introducing a needle into an interior of the capsule; puncturing a wall of the inhaler capsule by forcing the needle against the wall from the capsule interior to define the capsule outlet, moving the needle to de-obstruct the capsule outlet, and dispersing medicament from the inhaler capsule by directing compressed gas into the capsule from at least one element having a gas outlet positioned within the capsule. According to an embodiment of the invention, no more than 20% of the wall of the capsule displaced to form the capsule outlet is within the capsule interior after formation of the capsule outlet. According to some aspects, the gas is directed from at least two separate gas outlet positions within the capsule.

According to some aspects, the flow of gas is separately controlled for each of the at least two separate gas outlet positions.

According to an aspect, there is provided a method of expelling a dry powder medicament from an inhaler capsule, comprising: blowing gas through at least two gas outlets positioned within an interior volume of the inhaler capsule.

According to some aspects, delivery of the blown gas to the at least two outlets is separately controlled for each outlet.

According to some aspects, the method comprises allowing the gas and suspended dry powder medicament to leave the inhaler capsule through an outlet of the capsule.

According to an aspect, there is provided a method for actively delivering dry powder medicament contained in a capsule, the method comprising: introducing a needle having a gas outlet into an interior of the capsule; puncturing a wall of the capsule by forcing the needle against the wall from the capsule interior to define the capsule outlet; positioning the gas outlet of the needle within the capsule; directing compressed gas into the capsule from the gas outlet of the needle.

According to some aspects, the compressed gas is delivered from outlets in at least two different positions within the capsule in at least two flow regimes, and the capsule outlet is positioned so that the particles exiting the capsule outlet in a first flow regime are selected by the position of the capsule outlet to be of a different size or density than particles exiting the capsule outlet in a second flow regime.

According to some aspects, the compressed gas is delivered from outlets in at least two flow regimes, and the capsule outlet size is increased between the two flow regimes.

According to some aspects, the outlet size is increased by forcing the needle further into the capsule outlet than during the initial formation of the capsule outlet.

According to an aspect, there is provided a detachable module fittable to an inhaler for delivering dry powder medicament contained in a capsule, the module comprising a powder flow airway; wherein the powder flow airway is connectable to be in fluid communication with an airway of a base section of the inhaler; and wherein the powder flow airway comprises regions of the inhaler through which powder flows upon administration of the dry powder medicament.

According to an aspect, there is provided an inhaler for use with a detachable module fittable to an inhaler for delivering dry powder medicament contained in a capsule, comprising a gas flow airway connectable to be in fluid communication with a powder flow airway of a module through which medicament powder flows when the dry powder medicament is administered.

According to some aspects, the inhaler comprises a controller functionally connected to at least one from among the group consisting of a pressurized gas source, a gas flow sensor, and a valve.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

As will be appreciated by one skilled in the art, aspects of the present invention may be embodied as a system, method or computer program product. Accordingly, aspects of the present invention may take the form of an entirely hardware embodiment, an entirely software embodiment (including firmware, resident software, micro-code, *etc.*) or an embodiment combining software and hardware aspects that may all generally be referred to herein as a "circuit," "module" or "system." Furthermore, some embodiments of the present invention may take the form of a computer program product embodied in one or more computer readable medium(s) having computer readable program code embodied thereon. Implementation of the method and/or system of some embodiments of the invention can involve performing and/or completing selected tasks manually, automatically, or a combination thereof. Moreover, according to actual instrumentation and equipment of some embodiments of the method and/or system of the invention, several selected tasks could be implemented by hardware, by software or by firmware and/or by a combination thereof, e.g., using an operating system.

For example, hardware for performing selected tasks according to some embodiments of the invention could be implemented as a chip or a circuit. As software, selected tasks according to some embodiments of the invention could be implemented as a plurality of software instructions being executed by a computer using any suitable operating system. In an exemplary embodiment of the invention, one or more tasks according to some exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized for some embodiments of the invention. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, etc., or any suitable combination of the foregoing.

Computer program code for carrying out operations for some embodiments of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some embodiments of the present invention may be described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example, and for purposes of illustrative discussion of embodiments of the invention. In this regard, the description taken with the drawings makes apparent to those skilled in the art how embodiments of the invention may be practiced.

In the drawings:
FIG. 1 schematically illustrates a block diagram of the components of a portable active inhaler, according to some exemplary embodiments of the invention;
FIG. 2 schematically illustrates a partial view of some of the internal components of a portable active inhaler, according to some exemplary embodiments of the invention;
FIGs. 3A-3I schematically illustrate a capsule of medicament dry powder, pierced by the piercing needles of a portable active inhaler, and provided with compressed gas via the needles, according to some exemplary embodiments of the invention;
FIGs. 4A-4F schematically illustrate a portable active inhaler, according to some exemplary embodiments of the invention;
FIG. 5 schematically illustrates breathing rate curve, according to some aspects;
FIG. 6 schematically illustrates an exemplary method for operating an inhaler for delivering dry powder medicament during a single inhalation of the patient;
FIG. 7 schematically illustrates an exemplary method for operating an inhaler for delivering dry powder medicament in a prolonged manner over consecutive breathing cycles of the patient;
FIG. 8 schematically illustrates a method for delivering dry powder medicament to a user and piercing outward capsule outlets;
FIG. 9 schematically illustrates a method for delivering dry powder medicament to a user and producing various gas flow regimes within a dry powder medicament capsule;
FIGs. 10A-10C together comprise a schematic illustration of an exemplary method for operating an inhaler with a disposable head for delivering dry powder medicament during a plurality of inhalations of a patient;
FIGs. 11A-11D together comprise a schematic illustration of an exemplary method for operating an inhaler with a disposable head for delivering dry powder medicament during a single inhalation of a patient;
FIG. 12 shows an exemplary logged data summary sheet for an inhaler;
FIGs. 13A-13C schematically illustrate an inhaler with replaceable head, according to some exemplary embodiments of the invention; and
FIGs. 14A-14B show a cap for an inhaler in place and after removal, respectively.

### DESCRIPTION OF SPECIFIC EMBODIMENTS OF THE INVENTION

The present invention, in some embodiments thereof, relates to the field of dry powder delivery devices, and more particularly, to methods and systems for controlled delivery of dry powder.

### Overview

A broad aspect of some embodiments of the current invention relates to an inhaler for extracting dry powder medicament out of a capsule, and for delivering the medicament to a patient. In some embodiments, the inhaler includes a compressed gas source, at least one gas delivery lumen, at least one valve, at least one piercing needle, and at least one capsule chamber. The capsule chamber receives and holds a capsule containing dry powder medicament. The piercing needle and/or the capsule chamber are moveable with respect to each other *(e.g.,* the capsule chamber is moveable with respect to the needles, and/or *vice versa*). Optionally, the gas delivery lumen runs along (for example, axially within) the piercing needle. The valve regulates flow of compressed gas through the gas delivery lumen. In some embodiments, the gas lumen moreover includes at least one outlet for delivering gas into the capsule. In some embodiments, gas is delivered into the capsule from a pressurizing source (active device). In some aspects, gas is delivered into the capsule by capturing a portion of the pressure differential developed by patient inhalation (for example, patient inhalation creates a pressure differential across a capsule, inducing flow through a needle penetrating the capsule and out of an outlet of the capsule).

An aspect relates to determination of the flow of gas within a capsule used to release, disperse, and/or deagglomerate a dry powder medicament. In some aspects, a gas flow regime is administered within the capsule, wherein parameters of the flow regime relate to flow characteristics; for example: flow direction, gas velocity, trajectory, pressure, flow rate, and/or flow scheme (laminar or turbulent).

In some aspects, a regime of gas flow is chosen to reduce or remove the incidence of flow dead zones. For example, a chaotic and/or variable flow (such as flow from two or more separate flow regimes) is generated to help ensure that all regions of the capsule interior are swept by gas current. In some aspects, a regime of gas flow is chosen to create a preferred condition of separation. For example, a "centrifuging vortex" is optionally provided for all or part of a period of gas flow. Optionally, conditions are chosen (by location of the aperture relative to the capsule environment, and/or by restriction of shear forces/turbulence, for example) so that large particles (for example) are preferentially kept away from an exit aperture of the capsule, while smaller particles are allowed to exit. This differentiation is created, for example, by centrifuging action of a flow vortex, and/or by pulsed delivery of gas where there is a lag (of, for example, 10 msec, 50 msec, 100 msec, 200 msec, 500 msec, or another larger, smaller, or intermediate value) between relatively energetic gas injection (of, for example, 1 l/min, 0.5 l/min, 0.25 l/min, or another larger, smaller or intermediate flow rate) to a capsule (for deagglomeration), and a less energetic period of decompression (from an initially elevated pressure within the capsule due to pressurizing during gas injection) through a capsule aperture (leading to dispersal). In some aspects, there is switching between regimes suited to different functions. For example, an early regime is chosen to maximize deagglomeration and/or small particle dispersal (optionally, flow energy is increased for a particular flow volume; for example, multiple short bursts of high pressure gas are delivered rather than a lower pressure of more continuous gas delivery). In some embodiments, the burst duty cycle is about 50%. In some aspects, the burst duty cycle is about 10%, 25%, 66%, 90%, 100%, or another smaller or intermediate duty cycle.

In some aspects, later regime is chosen to maximize clearing of the capsule contents (for example, variation of flow direction and/or spatial source is increased, and/or capsule outlet aperture size is increased). In some aspects, switching between regimes is used to ensure clearing of residual powder: for example, a first flow regime is provided, and then a second (optionally a second and further) flow regime sweeps out regions of residual powder left by the first regime.

Optionally, gas flow regimes are produced by directing the compressed gas into the capsule from at least two spatially separated capsule gas inlets (alternatively described as needle or lumen outlets). Optionally, gas leaving the separated needle outlets is also separately controlled. Control comprises, for example, opening or closing (fully or partially) a valve gating one or more inlets, and/or regulation of a pressure differential which draws gas out of the needle outlet. For example, the gas can be directed through different gas lumens, different gas outlets of the same gas lumen, and/or a gas outlet can be moved for producing different gas flow regimes. Thus, different locations of needle outlets, and/or different combination of outlet locations, produce different gas flow regimes. Optionally, the gas flow regimes are modified by the shape of the gas outlets (e.g., a circular gas outlet or a slit-shaped outlet), the size of the outlets, and/or other outlet characteristics such as lumen diameter. For example, in some aspects, a needle lumen is about 1.0 mm in diameter. Optionally, the needle lumen diameter is about 0.5 mm, 0.75 mm, 1.25 mm, 1.5 mm, 2 mm, 2.5 mm, or another larger, smaller, or intermediate diameter. In some aspects, the lumen outlet diameter is, for example, about 0.5 mm. Optionally, the lumen outlet diameter is, for example, about 0.1 mm, 0.25 mm, 0.5 mm, 0.75 mm, 1.5 mm, or another larger, smaller, or intermediate diameter. Optionally, apertures of more than one size are used (for example, a greater flow is modulated by a smaller flow, the ratio of the two flows being set at least in part by the aperture ratio of 2:1, 3:1, 4:1, or another greater, smaller, or intermediate ratio).

In some aspects, the gas flow regimes are varied by regulating the gas flow rate via the gas outlets. For example, the gas is delivered continuously or in bursts (which duration and/or frequency are regulated), the gas can be delivered at different rates, pressures, velocities and/or amounts. Thus, by controlling the gas flow via the gas outlets, the outlet location, the outlet size and/or shape, and/or the compressed gas pressure, the gas flow regimes are further varied.

An aspect of some embodiments of the current invention relates to the directing of compressed gas into the capsule from spatially different locations within the capsule. Spatially different locations are, for example, locations in which the same aperture is moved (translated or rotated, for example) over time, and/or two or more apertures are provided. Spatially different locations are optionally distinguished, by axial position. For example: one location is more axially centered than another. Potentially, this affects the tendency and/or character of vortex flow within the capsule. In some embodiments, spatially different locations are distinguished by position relative to a capsule outlet (dispersal outlet). Potentially, a needle outlet into the capsule which is closer to a capsule outlet causes less initial dispersion of powder (for example, due to powder left "behind" it), than an outlet which is positioned more distantly from the capsule outlet. In some embodiments, compressed gas is directed into the capsule from at least two spatially separated gas outlets. Optionally, gas flow via the gas outlets is separately regulated. In some aspects, flow from a single outlet is varied over time by transitions of flow rate and/or outlet position. Optionally, the inhaler induces various gas flow regimes within the capsule, and switches between regimes. Potentially, application of turbulent and/or variable gas flow regimes and/or the combinations thereof reduce the amount of powder remaining trapped within the capsule, improve deagglomeration, and/or efficiently mix the powder particles. Optionally, the gas flow regimes and/or the combinations thereof are modified for different capsules, different powders and/or carriers, combinations of powders, different doses, patient characteristics (e.g., anatomy and/or breathing pattern), and/or different delivery patterns.

In some embodiments, the inhaler comprises a base section and a replaceable module which comprises functions and/or structures relating to the positioning of gas outlets in a capsule: for example, at least one needle comprising a flow lumen and flow outlet; a capsule chamber for containing the capsule, and/or mechanical and/or electrical means of placing the needle.

An aspect of the current invention relates to the preparation of a capsule so that it is suited to proper release of its contents (including effects on dispersal and/or deagglomeration) upon the introduction of gas flow therewithin. According to the invention, relative movement of the needle and capsule chamber during operation introduces a tip of the needle into a capsule. For example, this is by piercing a capsule inlet in a wall of a capsule held in the capsule chamber, punching a portion of the wall from the capsule, and/or otherwise using a member positioned within the capsule to create an aperture in the capsule wall. The piercing end (and/or end otherwise involved in capsule puncture) of the needle is then brought to form a capsule outlet at a wall of the capsule from within the capsule. For example, the needle is passed first into a proximal wall of the capsule, then out again at a wall distal to the initial piercing. Additionally or alternatively, the piercing portion of the needle changes direction during piercing; for example, the needle and/or its path is curved to penetrate and/or form apertures at adjacent wall portions.

In some embodiments, the inhaler comprises a base section and a replaceable module which comprises functions and/or structures relating to the formation of outlets in a capsule: for example, at least one needle comprising a sharpened or otherwise prepared tip for capsule puncturing; a capsule chamber for containing the capsule, and/or mechanical and/or electrical means of moving the needle.

This piercing of the capsule outlets from within the capsule and outwardly is referred to hereinbelow as intra-capsule piercing or outward piercing. Potentially, the outwardly extending flare (or other wall portion) of the capsule outlet reduces the amount of powder trapped in the capsule, and/or avoids formation of internal irregularities which could decrease the reproducibility of powder release and/or deagglomeration. The amount of wall portion left inside the capsule after capsule outlet formation is, for example, 0%, less than 5%, less than 10%, less than 20%, or less than another greater, lesser, or intermediate number. Potentially, more accurate prior determination of powder release and/or deagglomeration properties of an inhaler/capsule system is enabled by removing difficult to model and/or non-reproducibly formed irregularities from the capsule interior.

The effectiveness of dry powder medicament administration by dry powder inhalers is considered to be affected by numerous parameters beyond the simple amount of the active pharmaceutical ingredient (API) provided in a capsule. For example, small particles potentially reach deeper into the lungs, while large particles tend to deposit more superficially. However, even providing suitably smaller or larger particles during dose manufacture is potentially insufficient to achieve the desired dosing performance, since particles can also interact with each other and/or the capsule that contains them- they may clump or stick. Additionally or alternatively, carrier materials (such as lactose) are typically used, to provide desired medicament properties; for example, properties that assist in the manufacturability of a medicament. However, it may be further preferred that the carrier/API complex disassociate for dose delivery to a patient. Thus, an inhaler device is not only an administration device; it also performs aspects of last-stage processing of the medicament before delivery.

It is a potential advantage to open a capsule with a relatively small needle piercing, as this potentially allows the capsule to act for a longer period as a processing chamber for the powder which is to be administered. Potentially, it is an advantage to add high mechanical energy to the medicament powder while it is at its highest concentration, to promote a greater number of deagglomeration interactions (collisions). Additionally or alternatively, using an increased ratio of particle-particle interactions for deagglomeration is potentially preferred relative to the number of particle-wall interactions, to reduce loss of API along the inhaler flow path. Moreover, it is a potential advantage to concentrate wall/particle interactions that do occur over a relatively confined wall area, for example to decrease loss of API and/or to increase the chances of re-suspension of particles that do stick by subsequent collisions. According to the invention, a needle which pierces a capsule to provide an outlet is removed from the piercing, leaving an open hole in the capsule wall allowing the dispersal of medicament therethrough. In some embodiments, a portion of the piercing needle comprises a lumen with apertures locatable both inside and outside the capsule, allowing the needle to be positioned as a passageway for the dispersal of medicament from the capsule. This is a potential advantage, for example, to allow apertures to be provided which capture and then outwardly channel medicament from one or more locations within the volume of the capsule interior.

However, a potential advantage of using the pierced wall of the capsule directly to form an aperture is reduced concern that a needle passage will plug, particularly a needle which is to be used for the administration of a plurality of dosages.

An aspect relates to the coordinated creation of medicament flow outlet apertures and positioning of gas inlet apertures, to create a flow regime having properties which are controlled based on the relative features of gas inlets and medicament flow outputs. For example, flow is optimized for a particular ratio of rate of dispersal from the capsule to generation of disaggregating collisions within the capsule, based on the flow regime and the gas inlet characteristics chosen.

In some aspects, for example, a medicament flow outlet is positioned near the axis of the capsule, and a first (single-vortex, for example) flow regime is selected to rotate the medicament helically within the capsule. Potentially, the vortex flow of gas at least partially stratifies particles within the capsule, such that gas-borne particles reaching the medicament flow outlet are enriched in particles of a particular range of sizes, densities, and/or shapes. Optionally, a second flow regime interrupts this stratification, so that another distribution profile of particles reaches and exits the medicament flow outlet. In some aspects, the relative representation of size profiles of particles dispersed vs. particles in the capsule, in a particular flow regime, and for particles having at least a factor of 2 difference in size, is changed, for example, by at least 10%, 20%, 50%, 75%, or by another greater, lesser, or intermediate percentage. In some embodiments, the in-capsule/dispersed from capsule difference is changed in another particular flow regime by 25%, 50%, 75%, 100%, or another greater, lesser, or intermediate value.

In some aspects, characteristics of a gas flow regime are altered by changing the size, position and/or number of flow outlets in a capsule. For example, a medicament flow outlet is initially created small (for example, 0.5 mm in diameter), potentially increasing the average period of time during which a particle experiences high-energy (deagglomerating) conditions before being expelled from the capsule. After a first period of dispersal, the outlet configuration of the capsule is changed, in some embodiments, for example by puncturing or otherwise adding more outlets (1, 2, 3 or more capsule outlets, for example), increasing the size of an existing aperture (for example, by a diameter change of 25%, 50%, 100%, or another larger smaller or intermediate change), tearing wall material near the aperture, or otherwise modifying the capsule wall. Upon administration of a further gas flow regime, there is potentially a different profile of powder release; for example; with respect to distribution of particle sizes, and/or release efficiency.

An aspect of some embodiments of the invention relates to providing an inhaler with a removable (replaceable and/or disposable) module (a "head", but optionally a module of another type such as a cartridge) for use with a base. A potential advantage of this configuration is to allow a more expensive base system to be separated from components liable to contamination (with medicament powder, patient secretions, and/or moisture, for example). In some embodiments, the removable module comprises regions of the inhaler device which are liable to contact and/or contain powder. Such regions optionally comprise, for example, a capsule chamber, means for opening a capsule such as needles/blades, and/or portions of the device through which powder blows. In some aspects, the removable module comprises regions of the inhaler designed for patient contact, for example, a mouthpiece or mask.

In some aspects, a removable module comprises identifying information which relays identifying information about the module to the base. Potentially, this allows a base to be set up to reject operation with modules having the same mating parts, but carrying a different dosage. Additionally or alternatively, this allows a base to be set up to work with multiple dosage regimes-for example delivering different regimes of dispersal/deagglomeration gas, imposing different discharge threshold requirements, different status signaling, or another difference according to the preferred operating conditions for the identified module.

Before explaining at least one embodiment of the invention in detail, it is to be understood that the invention is not necessarily limited in its application to the details of construction and the arrangement of the components and/or methods set forth in the following description and/or illustrated in the drawings. The invention is capable of other embodiments or of being practiced or carried out in various ways.

### Overview of Inhaler Components and Use

### Components of an Exemplary Inhaler

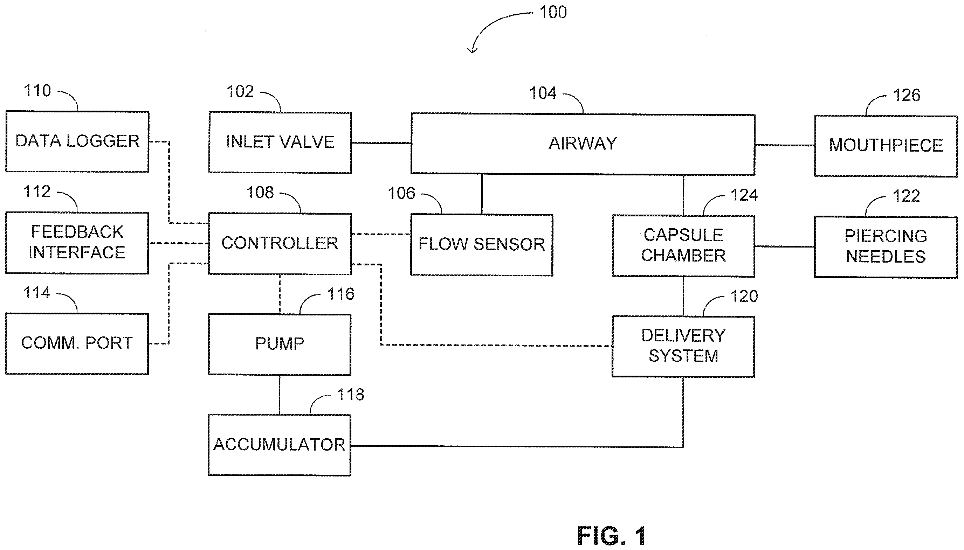

Reference is now made to *Figure 1**,* which is a schematic illustration of a block diagram of the components of a portable active inhaler **100,** according to some exemplary embodiments of the invention.

Before discussion of particular aspects of some embodiments of the invention, a brief introduction to elements of an exemplary inhaler **100** is provided.

In some examples, inhaler **100** includes an airway **104,** a gas delivery system **120,** piercing needles **122,** and a capsule chamber **124.** Medicament is delivered through a mouthpiece **126,** or optionally through a face mask or nasal adapter. In some embodiments, gas is pressurized by a pump **116** into an accumulator **118.** Optionally, another source of pressurized gas is used.

Apart from the gas pathway subsystem, some examples include means for sensing, control, monitoring and/or feedback. In some examples, the inhaler **100** further comprises a flow sensor **106,** a controller **108,** a data logger **110,** a feedback interface **112,** and/or a communication port **114.**

Optionally, in examples where appropriate corresponding elements are provided, various elements are in fluid connection with one another. For example: an inlet valve **102** and/or inlet restriction is provided in fluid connection with airway **104;** airway **104** is further in fluid connection with each of flow sensor **106,** capsule chamber **124** and/or mouthpiece **126;** pump **116** is in fluid connection with accumulator **118;** accumulator **118** is in fluid connection with gas delivery system **120;** delivery system **120** is in fluid connection with capsule chamber **124;** and/or capsule chamber **124** is in fluid connection with mouthpiece **126** via airway **104.** Control coupling includes, for example: controller **108** coupled with flow sensor **106,** data logger **110,** feedback interface **112,** communication port **114,** pump **116,** and/or with gas delivery system **120,** for controlling the operation thereof.

Airway **104** is a fluid passageway that allows passage of fluids to and from mouthpiece **126.** Optionally, valve **102** determines the restriction of flow through airway **104,** and/or restriction is determined by the form of the airway **104** itself (for example, a form including a restriction in the cross-section of the airway). It is noted that the airway restriction potentially affects drug deposition. Optionally, inlet valve **102** is a one way valve *(i.e.,* a check valve) that also prevents exhaling into inhaler **100.**

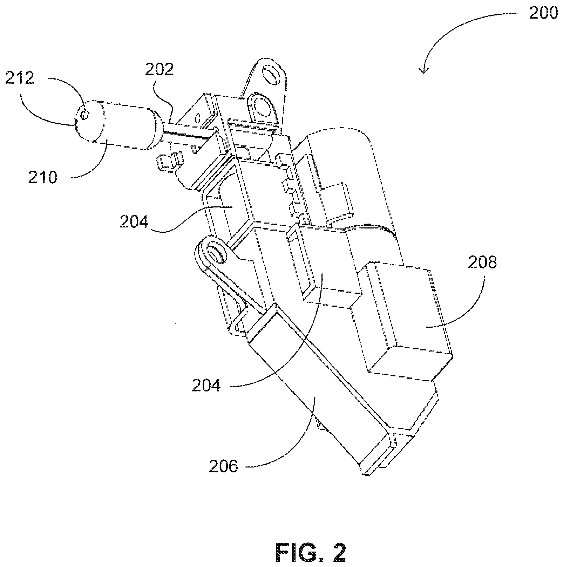

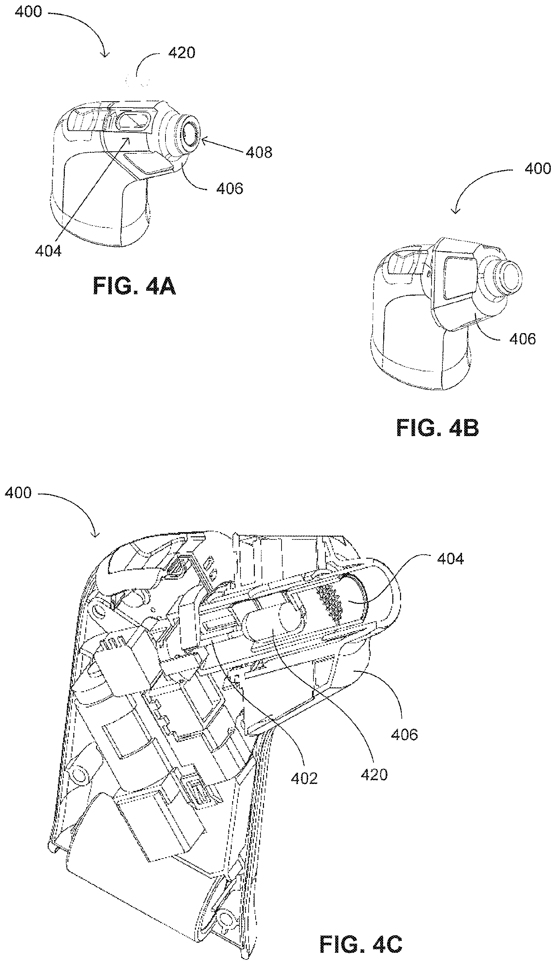

In some embodiments, inhaler **100** is a portable hand held inhaler as can be seen, for example, in relation to inhalers **200** and **400** of *Figures 2* and *4A**-4F,* respectively.

In some examples, the inhaler is a self-powered inhaler including an internal power source, such as a battery for providing power to the various components of the inhaler, such as the controller **180,** the flow sensor **106,** the gas source **116, 118,** and/or the gas delivery system **120.** Alternatively, the inhaler can be coupled with an external power source, either for recharging an internal battery or for directly powering the inhaler.

### Exemplary Stages of Inhaler Use

In some examples, use begins with the user loading a dry powder medicament capsule into capsule chamber **124.** The user arms and sets up inhaler **100,** preferably in a single maneuver (e.g., by rotating a priming knob), as also described hereinbelow with reference to *Figures 4A-4F**.* The arming and setting up maneuver includes all or part of the following actions:
- Optionally, pump **116** pumps gas into accumulator **118,** and/or another pressure source is optionally otherwise prepared and/or verified for use. Alternatively, accumulator priming is postponed until powder delivery.
- In some embodiments, capsule chamber **124** is moved toward needles **122** such that needles **122** pierce capsule inlets in the proximal wall of the capsule. Optionally, the needles are moved toward the capsule chamber.
- Capsule chamber **124** and needles **122** are further moved such that needles **122** penetrate into the capsule via the pierced inlets and pierce capsule outlets from within the capsule.
- Following piercing of the capsule outlets, the needles optionally retract to position the gas outlets of the gas lumens in the needles within the capsule for delivering the compressed gas into the capsule.

Use, in some examples, continues with the patient bringing mouthpiece **126** to the mouth and taking a deep breath. In some embodiments, when the inspiratory flow rate exceeds a threshold, controller **108** opens the valves of delivery system **120** for delivering the compressed gas into the pierced capsule, which is optionally accompanied by activation of pump **116.** Delivery of compressed gas deagglomerates, disperses, mixes, and releases the dry powder medicament toward the patient. Alternatively, in case of employing a face mask instead of a mouthpiece, the patient attaches the face mask to the face and breathes in and out through the face mask, so that flow sensor **106** can acquire flow readings. Controller **108** optionally calculates breathing parameters of the patient, and provides breathing feedback to the patient via feedback interface **112** *(e.g.,* by blinking a LED, and/or by an audio beep) that inhaler 100 is ready for action.

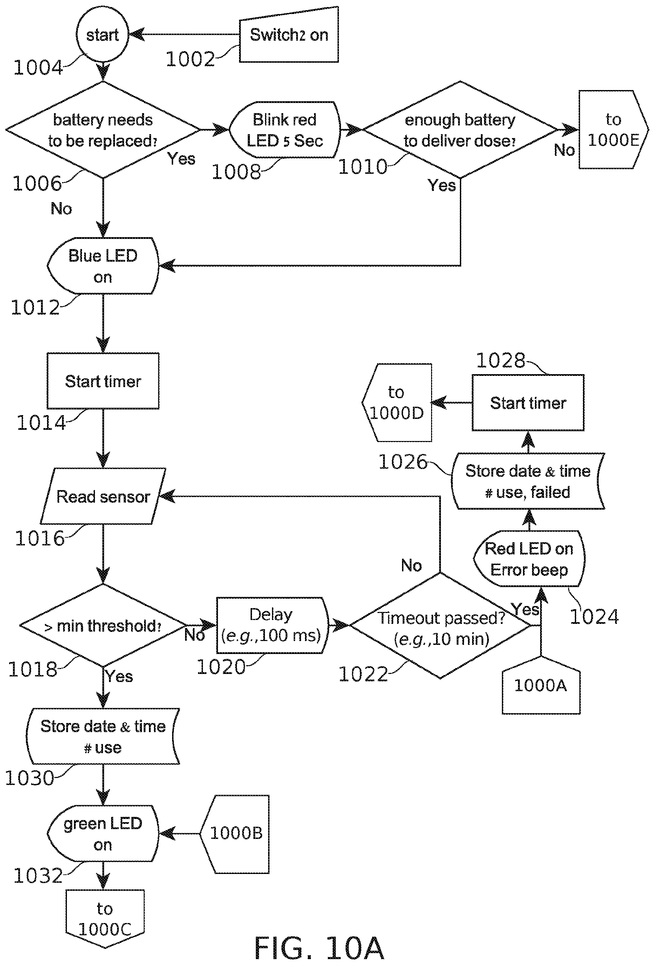

In some examples, a facemask or nasal adapter is used. Optionally, such interfaces are used with a prolonged (multiple inhalation) delivery paradigm. In a multiple inhalation paradigm, each time the sensor detects patient inhalation, a portion of the capsule drug content is dispersed. For example a system is optionally set to deliver a fixed burst of medicament laden air over a number of inspirations (for example, 5, 10, 20, or another greater, larger, or intermediate number of inspirations). Optionally, a feedback interface (such as a LED illumination and/or sound indication) indicates dug delivery status, for example as described in relation to *Figures 10A-10C* hereinbelow. Delivery repeats at appropriate inhalation points until the capsule is empty. In some embodiments, the device controller tracks how much drug is dispersed over time and/or delivery cycles, allowing determination of the empty capsule condition). Optionally, when the capsule is empty the feedback interface indicates complete delivery. Optionally, the portion of the dose that is delivered per respiration, and/or the phase of the respiration is the same or different for each inhalation, according to the settings of the controller. A potential advantage of dividing a dose this way is to increase the reliability of dose delivery. Additionally or alternatively, it allows making individual deliveries smaller, and/or to allow a larger powder load (likely to trigger a reflex cough, for example, if delivered all at once) to be divided among multiple inhalation cycles.

Controller **108** controls the valves of delivery system **120** for delivering the compressed gas into the capsule, and for releasing thereby the dry powder medicament toward the patient, in coordination with the breathing of the patient. It is noted however, that the inhaler optionally monitors the patient respiratory cycles and breathing parameters through the mouthpiece or through the nasal adapter, as well as through the facemask. Additionally, an inhaler with a mouthpiece or with a nasal adapter can also be employed for drug delivery over several breathing cycles.

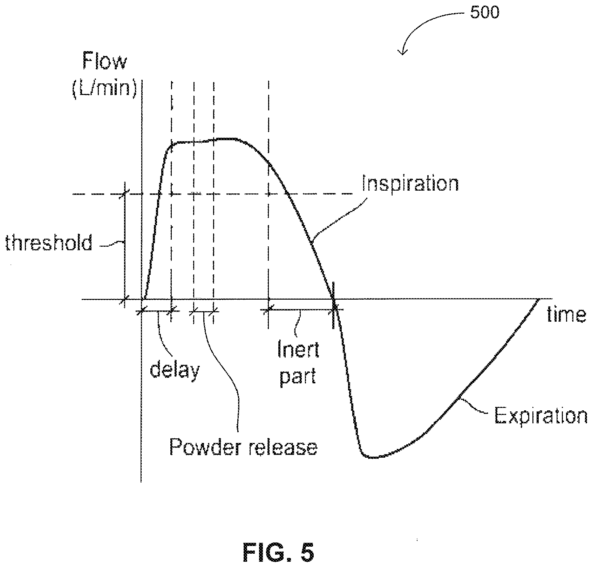

In some examples, inhaler **100** coordinates and controls the delivery of the dry powder medicament according to a release condition. The release condition can be associated with, for example, the inspiratory phase, and/or an inspiratory flow rate threshold (described, for example, in relation to *Figure 5* hereinbelow), and/or with the expiratory phase and/or flow rate. Other release conditions can relate, for example, to the carbon dioxide content, the oxygen content, the moisture content, the pressure of the inspiratory phase, the blood glucose level and/or other blood content parameters. Still other release conditions relate to a secondary effect; for example, a side effect of the dry powder medicament. For example, morphine delivery to a patient is not released until blood pressure exceeds a threshold. Other release conditions relate to the time of day or to other measurements, such as the heart rate of the patient.

Additionally or alternatively, the release of the dry powder is controlled manually. In this manner, the user *(e.g.,* the patient, a physician or a caretaker) triggers delivery, for example, by pressing a drug release button. The user optionally triggers drug delivery according to the readings of the flow sensor or according to other (or additional) sensors, such as a blood pressure sensor and/or glucose blood level sensor.

### Active Gas Flow Regimes

In some examples, gas flow regimes are actively produced by the inhaler **100** within the capsule for preparing the dry powder for delivery (e.g., deagglomeration and dispersion), for extraction of the dry powder from its' capsule and for delivering the medicament to the patient. Optionally, gas flow regimes are adapted, for example: to the powder to be deagglomerated and dispersed, the carrier, the patient (e.g., anatomy and breathing), and/or the target region for the medicament.

In some examples, elements involved in determining the gas flow regime include the gas delivery system **120,** piercing needles **122,** capsule chamber 124, and the capsule itself (for example capsule **210, 300,** and/or **420** of *Figures 2**,* *3A-3G**,* and/or *4C-4E**,* respectively). Gas is sourced, for example, from a pump **116** and/or accumulator **118,** or from another source of pressurized gas. Operation of the flow system is under the control of a controller **108,** optionally modified based on inputs from one or more sensors such as flow sensor **106.**

In some embodiments, compressed gas is delivered into the capsule via delivery system **120.** Delivery system **120** delivers the compressed gas into the capsule for deagglomerating, aerosolizing, and/or releasing the dry powder medicament from the capsule. In some embodiments, the delivery is direct, for example, via at least one gas delivery aperture introduced to the interior of the capsule. Optionally, the delivery aperture is an aperture of needle **122.** In some examples, system **120** enables inhaler **100** to produce a plurality of gas flow regimes within the capsule.

In some examples, flow is delivered to the capsule at a rate of about 1 liter per minute (1 l/min). In some examples, a peak rate of gas flow is, for example, about 10 l/min, for example, during release of pressure previously stored in an accumulator by a pump. In some examples, the maximum gas flow (such as during a valve opening with full pressure developed by the system's gas source) is, for example, about 0.1 l/min, 0.5 l/min, 1 l/min, 2 l/min, 5 l/min 10 l/min, 20 l/min, or another greater, lesser or intermediate flow rate. In some examples, the flow rate is continuously adjustable from no flow up to the maximum flow rate. In some examples, flow rate is adjustable in a number of steps. Control of flow rate comprises, for example, setting a valve opening time, a valve opening fraction, an inlet aperture size, an outlet aperture size, a gas source pressure, a gas source operating time, and/or another parameter.

In some examples, the maximum pressure developed by a pump is, for example, about 500 mmHg above gauge pressure. In some examples, the pressure developed is another number, for example, 50 mmHg, 100 mmHg, 200 mmHg, 400 mmHg, 800 mmHg, or another greater, lesser, or intermediate pressure.

### Features, Parameters, and Uses of Active Gas Flow Regimes

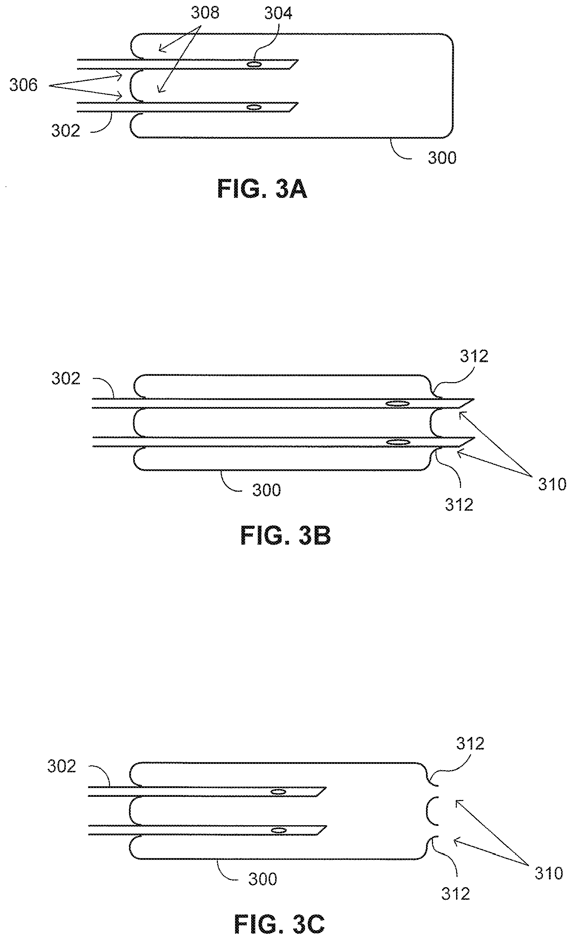

Parameters of a gas flow regime include, for example: gas flow direction, gas velocity, gas trajectory, the amount of compressed gas directed into the capsule, the pressure at which the gas enters the capsule, the cycle of pressure experienced within the capsule, and/or parameters of pulsed or continuous flow. In some examples, one or more of these parameters is modified, for example, by delivering the gas via at least two separately controlled gas outlets, by controlling the gas pressure within the accumulator (or other delivery system pressure), by regulating flow of gas via the outlets, and/or by adapting the outlet shape, size, and/or position. The control of the gas flow within the capsule is elaborated on hereinbelow with reference to *Figures 3A-3G**.*

In general, the inventors have found that removal of residual powder is significantly increased when at least two flow regimes are used to disperse powder, each administered from a at least one outlet having a different position and/or direction of flow than in the other regime. A two-needle system potentially accomplishes this without the need for additional motion of the system during ventilation (apart from that to independently drive the valves which gate the flow of gas). However, in some embodiments, a single needle is used, and the two-position condition is met by moving the needle (for example, advancing, retracting, and/or rotating the needle).

Dry powder inhalers use gas suspension to transfer dry powdered medicament to the target organ (typically the lungs). One method of packaging the medicament is within a capsule. Typically, an inhaler punctures the capsule to allow a flow of gas to reach the capsule contents. Active inhalers provide an active pressure source for achieving gas suspension. This provides a potential advantage over inhalation-driven powder dispersal by removing at least some variability in drug delivery due to patient performance. For example, Kaufmann et al. (WO2011/077414) describe a system which punctures the capsule to deliver gas flow directly to the capsule interior.

Conventional capsules can include various medicament dry powders with various active ingredients, and optionally include various inert carrier materials. The medicament powder (and the carrier) should sometimes be deagglomerated prior to delivery to the patient. Furthermore, different patients or different treated target sites may benefit from different levels of deagglomeration. For example, children may require more extensive deagglomeration *(i.e.,* producing smaller powder particles) than adult patients. Medicament which is delivered to the upper regions of the breathing system may be less deagglomerated that medicament delivered to the deepest regions of the lungs. In case of delivering more than a single powder material (e.g., from different capsules, or from a single capsule), each medicament powder may be better deagglomerated by different gas flow regime or combination of regimes. Therefore, controlling the deagglomeration of the dry powder can improve the treatment.

In some examples of the present invention, appropriate control of gas flow (by one or more appropriately selected flow regimes) is used to reduce residual powder.

The dynamics of the gas flow used to suspend and/or transport a dry powder medicament can have an effect on the performance of the inhaler device. For example, some of the dry powder medicament, intended to be delivered to the patient, might be trapped within the capsule (this trapped powder which should have been delivered, is also referred to herein as residual powder). This reduces delivery efficacy (which can be defined as the amount of powder delivered divided by the amount of powder that should have been delivered according to the treatment plan). Furthermore, the amount of undelivered residual powder might change for different capsules, even similar capsules, and thus decreases dose uniformity. Thus, reduction of residual powder provides a potential advantage to ensure that the patient received the prescribed dose (and/or for monitoring the dose delivered to the patient), for maintaining dose uniformity, and/or for reducing medicament costs.

In some examples, appropriate control of gas flow (by one or more appropriately selected flow regimes) is used to control deagglomeration.

API particles can have some tendency for self-adherence, which potentially changes transport and/or absorption properties of the ingredient. Additionally or alternatively, medicament is preferentially provided in a relatively coarse form for capsule manufacturing (for example, API attached to a carrier such as lactose), but preferentially delivered to the patient's respiratory tract in finer form (for example, with API separated from the carrier).

### Examples of Active Gas Flow Regimes

The following describe other examples for which flow regime adaptability provides a potential benefit:
- In some examples, a flow regime is configured to provide bursts which accommodate a breathing pattern of a patient. For example, a patient who is capable of deep inspirations is provided with an inhaler that gives a prolonged series of flow bursts (several hundred milliseconds up to several seconds, for example), allowing administration of the contents of a medicament capsule within substantially a single breath. Alternatively, a patient is provided with a brief burst during each of a series of inspirations (typically spaced over several seconds and up to a minute or two), allowing drug delivery to a patient without a requirement for the patient to alter their normal breathing pattern.
- In some examples, a combination of different flow regimes is employed for improving extraction of the dry powder medicament from the capsule and significantly reducing the residual powder. Potentially, the use of different flow regimes helps to avoid a situation where powder accumulates in a particular region of the capsule, and/or a particular size of medicament particle is retained within the capsule.
- In some examples, a first medicament powder (or mixture of powders) is deagglomerated better by a first gas flow regime, and a second medicament powder is deagglomerated better by a second gas flow regime. For example, a formulation that is based on API and lactose carrier is dispersed under a flow regime that efficiently separates between the API and the carrier; a formulation that contains only active ingredients (e.g. proteins) and no carrier particles is dispersed under a different flow regime that optimally deagglomerates the specific powder.
- In some examples, there are several powders, and different gas flow regimes aid in mixing of the powders.
- In some examples, different flow regimes are used to enable desirable drug delivery for different drug doses or different patient inhalation patterns (e.g. tidal breathing or single deep inhalation).
- In some examples, different regimes are employed for deagglomerating the dry powder for producing different average particle sizes *(e.g.,* a first combination of regimes for producing a first average particle size, and a second combination of regimes for producing a second average particle size). Optionally, for delivering the drug to the upper regions of the respiratory system of the patient, the drug is deagglomerated to relatively large particles as produced by a first gas flow regime. Optionally, for delivering the drug to the deepest regions of patient's lungs, the drug is deagglomerated to relatively small particles produced by a second or further gas flow regime.
- In some examples, a first gas flow regime is selected to deagglomerate particles, optionally at the expense of efficiently expelling them from the capsule. For example, a gas escape aperture is placed where particle concentrations are low in the first flow regime. Potentially, deagglomeration takes place more efficiently within the capsule where particles are concentrated, and mechanical energy delivery is focused. After the deagglomeration regime, a second regime is used to help ensure particles *are* expelled, so that little or no medicament is left in the capsule.
- In some examples, a flow regime is selected to preferentially expel medicament during a particular phase of the respiratory cycle; for example, to target deeper or upper respiratory tissues. Additionally or alternatively, the flow regime is adapted to even the rate of medicament expulsion. For example, flow turbulence is increased (optionally while flow remains about the same) as the amount of remaining medication decreases. Optionally, the increase is synchronized with respiratory phase. Potentially, this helps to even out the distribution of medicament through the respiratory tract.

### Plurality of Gas Delivery Apertures for Dispersal and Deagglomeration

In some embodiments, delivery system **120** delivers the compressed gas from at least two spatially separated locations within the capsule. For example, the delivery system includes at least two outlets (optionally, outlets of one or more needles **120)** for directing the compressed gas into the capsule. Additionally or alternatively, the delivery system includes an outlet which can be moved within the capsule (for example, advanced, retracted, and/or rotated) for producing different gas flow regimes.

In some exemplary embodiments of the invention, delivery system **120** includes two gas lumens. Each lumen includes at least one gas outlet *(e.g.,* a side hole in the gas lumen). In operation, each lumen penetrates the capsule to position the gas outlet thereof within the capsule. The gas outlets of the gas lumens are thus located at spatially separated locations within the capsule.

Optionally, each gas lumen is associated with a respective gas valve (and/or other pressure control means) to regulate the compressed gas flow therethrough. Thereby, the gas is delivered from two spatially separated locations *(i.e.,* the locations of the gas outlets), which are also separately controlled.

In this manner, a plurality of gas flow regimes can be produced within the capsule, for example, as described hereinbelow with reference to *Figures 3A-3G**.* Examples include: a first gas flow regime in which a first lumen delivers gas, and a second lumen is shut; a second regime in which the first lumen is shut, and the second lumen delivers gas; and a third regime in which both lumens deliver compressed gas into the capsule simultaneously. As mentioned above, by employing a plurality of gas flow regimes the amount of residual powder is potentially decreased. For example, a powder particle which is trapped inside the capsule and is not released when gas flows according to the first gas regime, might be released by the second or the third regimes.

In some embodiments, each valve of each gas lumen is operable to select from among a plurality of flow levels *(e.g.,* a valve can have flow levels of fully open and partially open, in addition to shut). Thereby, inhaler **100** can produce additional gas flow regimes within the capsule; for example: a first regime in which the first valve is fully open and the second valve is partially open, a second regime in which the first valve is fully open and the second valve is shut, a third regime in which the first valve is partially open and the second valve is fully open, a fourth regime in which the first valve is shut and the second valve is fully open, and/or other regimes according to other combinations available from the settings of the valve or valves.

In the example set forth in *Figure 2**,* hereinbelow, a single gas source is coupled to two lumens via two respective valves. However, it is noted that for producing a plurality of gas flow regimes, embodiments of the delivery system optionally include different number of gas lumens, each lumen optionally includes different number of gas outlets, and/or outlets are optionally of different shape and size. Embodiments of delivery system **120** optionally include different numbers of valves, each valve optionally regulates fluid flow in a different number of lumens, and/or each valve optionally has a plurality of flow levels. Alternatively or additionally, each lumen or group of lumens is optionally coupled to a different gas source, which optionally stores a different gas or that stores gas at a different pressure.

### Control of Capsule Inlet and/or Outlet Formation

According to the invention, piercing needles **122** pierce the capsule in capsule chamber **124** for producing capsule inlets. The capsule inlets enable delivery system **120** to penetrate the capsule for delivering the compressed gas thereinto.

In some embodiments, the gas lumens of delivery system **120** are incorporated within piercing needles **122** (for example, as lumens surrounding the axial center of the needle). This coordinates the piercing of the capsule inlets by needles **122** with penetration of the gas lumens into the capsule.

For release of gas, needle(s) optionally have a side hole, and/or a hole at the distal end thereof. A potential advantage to a side aperture in contrast to a distal end aperture is to avoid plugging of the gas aperture by capsule material during penetration. The two types each offer different flow patterns, and are optionally used together and/or alternately. According to the invention, the piercing needles **122** also are operable to produce outlets. The outlets are produced by piercing from within the capsule interior. The capsule outlets allow the released dry powder medicament to exit the capsule toward airway **104** and mouthpiece **126.**

When operated from within the capsule, piercing needles **122** produce the capsule outlets such that the flare of capsule material that surrounds each outlet extends outwardly from the capsule, as described in relation to *Figures 3A-3G* hereinbelow. In other words, the ruptured walls that surround the capsule outlet (these ruptured wall portions are referred to herein as the flare of the capsule outlet or aperture) extend outwardly from the capsule. A potential advantage of this configuration is that the flare is positioned where it avoids trapping powder particles within the capsule.

In accordance with an exemplary embodiment of the invention, piercing needles **122** include at least one needle (not shown) for forming inlets and outlets within the capsule. The piercing needles are constructed (e.g., in terms of structure and materials) such that they can withstand repeated piercing of replaceable capsules; for example, the needles are constructed of stainless steel. To bring about piercing, at least one of capsule chamber **124** (holding the capsule) and piercing needles **122** is moveable with respect to the other. Thus, the capsule is moved toward piercing needles **122** (or *vice versa)* until each needle pierces a capsule inlet in the capsule, and, in some embodiments, motion continues or resumes until each needle (and/or at least one of the needles) pierces a capsule outlet from within the capsule. In this manner, the capsule outlet is pierced from within the capsule, and thereby, the flare of the capsule outlet extends outward from the capsule.

In some embodiments of the invention, a piercing needle **122** is of two or more gauges, having varying diameter (and possibly varying circumferential shape) along the needle. The variation may be discrete (two different two diameters, for example), or continuous (for example, tapering). By this, the diameter of the capsule inlets and outlets is optionally controlled. For example, the capsule outlet is made smaller than the respective inlet.

In accordance with another example, the capsule outlet produced for a first powder material is produced to be of a first shape and size by moving the needle through the outlet until a first section of the needle (having a first cross section) produces the desired outlet. The outlet produced for a second powder material is produced of a second shape and size by moving the needle through the outlet until a second section of the needle (having a second cross section) produces the desired outlet.

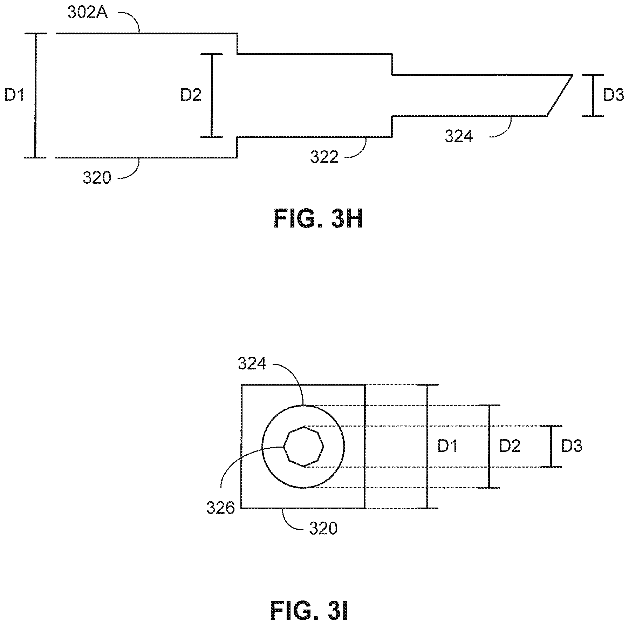

In another example, an outlet aperture is first made small during an early phase of medicament release, and then widened during a later phase of medicament release. Potentially, this helps to regulate the ratio of deagglomeration to release. For example, the early phase, where the aperture is small, contains the powder so that it is exposed to more of the deagglomerating energy of the compressed gas. The later phase, where the aperture is large, helps to reduce the amount of residual powder by making it more likely for the powder to escape the capsule. The two phases optionally occur during a single breath, and/or are spread over two or more breaths. An exemplary needle structure is detailed further herein below with reference to *Figures 3H* and *3I**.*

In some embodiments, piercing needles **122** include needles of different lengths. In this configuration, the number of outlet holes can be less than the number of the inlet holes, as the short needle(s) will not reach the distal surface of the capsule during the piercing process.

Exemplary forms of the tip of each of the needle include a cone shaped tip, a beveled tip of various inclination angles, or any other shape suitable for penetration of the capsule material. Use of a side aperture potentially also allows the tip of the needle to be designed for penetration only; for example, as a cone-shaped tip. It is also noted that a cone shaped tip needle creates an approximately circumferential flare surrounding the pierced aperture, while a beveled tip needle tends to create a cut piece *(i.e.,* the piece of the capsule wall cut out by the beveled tip) extending from the aperture.

As mentioned above, in accordance with an embodiment of the disclosed technique, the gas lumens of delivery system **120** are incorporated into piercing needles **122.** For example, each of piercing needles **122** includes therewithin a gas lumen ending at a gas outlet. In this manner, the needles pierce the capsule inlet, penetrate therethrough into the capsule and continue to pierce the capsule outlet. Thereafter, the needles are retracted such that the gas outlet of the gas lumen is located within the capsule.

Capsule chamber **124** is a chamber for holding a dry powder medicament capsule. As mentioned above, capsule chamber **124** may be moveable with respect to piercing needles **122** by an actuating mechanism (not shown). Alternatively, capsule chamber **124** is immobile and needles **122** are moveable. Further alternatively, both capsule chamber **124** and needles **122** are moveable and can both move back and forth, one toward the other. Optionally, each of needles **122** can be moved separately with respect to capsule chamber **124.**

In some embodiments of the invention, capsule chamber **124** is adapted to hold conventional capsules *(i.e.,* the capsules are not specially adapted to capsule chamber **124,** but are capsules of standard size). Optionally, capsule chamber **124** is made to suit one or more of several sizes of capsules, or to be exchangeable and/or selectable for different sizes. Thus, the capsule can be an 'off the shelf' or generic capsule, such as capsules manufactured for use with common types of dry powder inhalers. The capsule can be, for example, a gelatin or **HPMC** capsule, a blister and/or foil, a capsule made from a single material with no foil.

Additionally or alternatively, capsule chamber **124** can also hold custom-made capsules *(i.e.,* as opposed to conventional, off the shelf, capsules), designed for specific purposes or devices.

In some embodiments, capsule chamber **124** is sized to hold several capsules at the same time. Additionally or alternatively, inhaler **100** includes more than a single capsule chamber.

Optionally, for simultaneous delivery of powders stored in a plurality of capsules, each capsule is pierced by different needle(s) **122,** and is provided with compressed gas via different lumen(s). In some embodiments, loading of the capsule into capsule chamber **124,** and extracting the capsule from within capsule chamber **124** is performed manually and/or individually. Alternatively or additionally, a magazine or cartridge of capsules is loaded into the inhaler and feeds the inhaler capsules as required-one at a time, or in sets as appropriate. In set-loaded inhaler configurations, several medicaments or doses can be deagglomerated, aerosolized, released, and/or delivered together.

In case capsule chamber **124** holds more than a single capsule, piercing needles **122** pierce at least one of the capsules. Inhaler **100** determines which capsules to pierce according to the medicament powder to be delivered, the dose of powder to be delivered, the capsule loading order *(i.e.,* in case the inhaler is loaded with a cartridge of capsules), or according to other considerations or a combination of the considerations listed above.

It is noted that the capsules loaded into capsule chamber **124** can be independent capsules *(i.e.,* a single unlinked capsule) or physically linked capsules. Linked capsules are a group of capsules physically connected such that they form together a continuous chain of dry powder compartments. Thus, for example, inhaler **100** can be a disc (or square) type dry powder inhaler receiving powder disc into capsule chamber **124.**

### Production of Different Gas Flow Regimes within the Capsule

Reference is now made to *Figures 3A-3I**,* which are schematic illustrations of a capsule of medicament dry powder **300,** pierced by the piercing needles **302** of a portable active inhaler, and provided with compressed gas via gas outlets **304** of gas lumens, according to some exemplary embodiments of the invention.

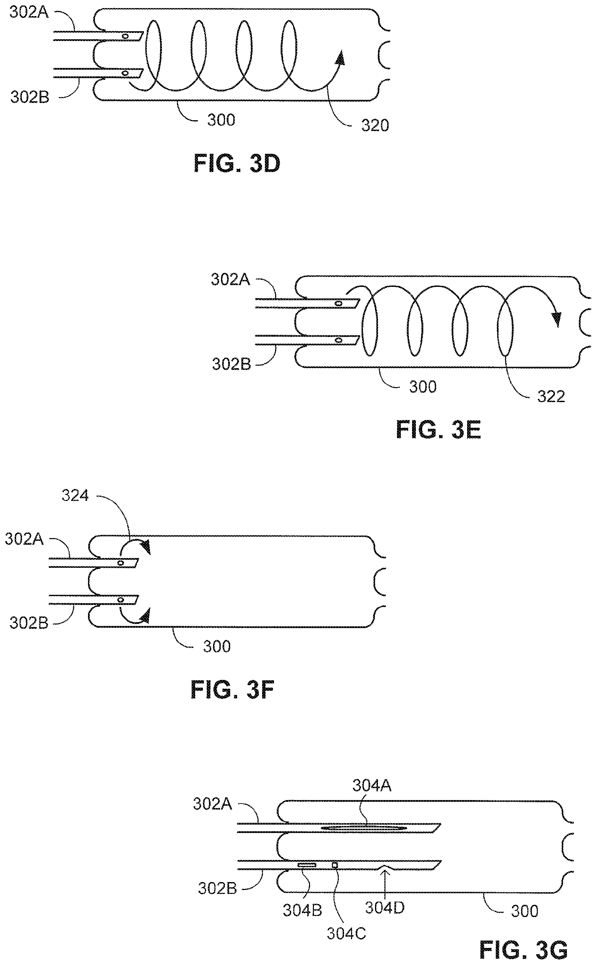

*Figure 3A* depicts capsule **300** after the piercing needles have pierced a pair of entrance apertures in the walls thereof. *Figure 3B* depicts capsule **300** after the piercing needles have additionally pierced a pair of exit apertures **310** in the walls thereof. *Figure 3C* depicts capsule **300** after the piercing needles have retracted such that the outlet ports of the fluid lumen within the capsule are positioned within the capsule, and the exit apertures **310** are clear for the outflow of gas from the capsule. *Figure 3D* depicts a first gas flow regime within capsule **300.** *Figure 3E* depicts a second gas flow regime within capsule **300.** *Figure 3F* depicts a third gas flow regime within capsule **300.** *Figure 3G* depicts various exemplary gas outlets. *Figure 3H* depicts the external shape of needle **302A** from a side view perspective. *Figure 3I* depicts the external shape of needle **302A** from a top view perspective.

With reference to *Figure 3A**,* capsule **300** is held within a capsule chamber of an inhaler (chamber positioning of the capsule is discussed in relation to *Figures 4A-4F* hereinbelow). Once the user loads capsule **300** within the capsule chamber and primes the inhaler, the capsule chamber is moved toward needles **302** until the needles pierce respective capsule inlets **306** in the wall of capsule **300** proximal to the needles. As can be seen in *Figure 3A**,* when piercing capsule inlets **306,** needles **302** produce a flare of the capsule wall extending in the direction of the piercing direction. That is, when piercing inlets **306** from outside capsule **300,** flares **308** extend inwards into capsule **300.** It is noted that flares **308** extend inwards into capsule **300** and embrace the needles. Thereby, the proximal side of capsule **300,** in which entrance apertures **306** *(i.e.,* capsule inlets **306)** are pierced, has no corners, and thus powder is easily extracted from capsule **300** and the amount of residual powder is decreased.

With reference to *Figure 3B**,* the capsule chamber further moves toward needles **302** until the needles pierce respective capsule outlets **310** in the distal wall of capsule **300.** As can be seen in *Figure 3B**,* when piercing capsule outlets **310,** needles **302** produce a flare of the capsule wall extending in the direction of the piercing direction. That is, when piercing outlets **310** from within capsule **300,** flares **312** extend outward from capsule **300.** Thereby, flares **312** do not trap any dry powder particles within capsule **300,** thus decreasing the amount of residual powder that is not extracted from capsule **300.**

With reference to *Figure 3C**,* the capsule chamber retracts away from needles **302,** such that capsule outlets **310** are clear *(i.e.,* unblocked by needles **302**)**,** and such that gas outlets **304** of needles **302** *(i.e.,* of the gas lumens incorporated within needles **302**) are positioned within capsule **300** for delivering compressed gas into capsule **300.**

The needles of the inhaler device deliver the compressed gas into the capsule. The inhaler device can produce several gas flow regimes within the capsule. By employing a plurality of gas flow regimes, the inhaler potentially decreases the amount of residual powder remaining trapped within the capsule. Furthermore, the various gas flow regimes enable the inhaler to deagglomerate, aerosolize, mix and release various dry powder materials which may differ in the size of the particles, and other parameters of the dry powder and of the potential carrier material within the capsule. Additionally, different regimes (or combinations of regimes) can be used for deagglomerating and dispersing different mixtures of materials, and can further be used for producing particles of different sizes *(e.g.,* for delivering the powder to different body locations).

*Figures 3D-3F* depict three exemplary gas flow regimes produced by pair of needles **302.**

With reference to *Figure 3D**,* gas flow regime **320** is produced within capsule **300** by delivering compressed gas from a needle **302A,** while keeping a needle **302B** shut. It is noted that the gas path *(i.e.,* trajectory) within capsule **300** is similar to a counterclockwise helix. With reference to *Figure 3E**,* gas flow scheme **322** is produced within capsule **300** by delivering compressed gas from needle **302B,** while keeping needle **302A** shut. It is noted that the gas path within capsule **300** is similar to a clockwise helix. With reference to *Figure 3F**,* gas flow regime **324** is produced within capsule **300** by delivering compressed gas from both needle **302A** and needle **302B.** Potentially, this gas flow regime **324** is turbulent. It is a potential advantage to administer two or more (for example, all three) of these flow regimes during dispersal of a medicament. For example, if static zones allowing powder buildup are set up by a clockwise helix, a strong-flowing counterclockwise helix has the potential to disrupt the pattern that allowed this deposition, re-suspending powder from the former region of static flow (and vice-versa). A turbulent flow potentially leaves no constant static region of powder accumulation behind, but also potentially does not develop the highest gas velocities for resuspension/deagglomeration. Thus, the mix of high turbulence, and high, alternating-direction velocities potentially covers a range of conditions which together serve to sweep the great bulk of residual powder out of the capsule.

Optionally, the needles **302A, 302B** are positioned with one more eccentric to the capsule's central axis than the other. Optionally, the needles **302A, 302B** are positioned with one further advanced along the capsule length than the other. Potentially, this allows setting up axially or otherwise spatially separated patterns of flow, for example, regions of higher and lower pressure, regions separated by a layer of difference in flow velocity, or another combination of flow patterns. Optionally, the flow patterns generated are selected for control of particle motion between them; for example, a boundary optionally impedes (or encourages) motion across itself, and/or optionally selectively impedes (or encourages) crossing of larger, smaller, denser, and/or less-dense particles.

For further varying the gas flow regimes and for producing additional gas flow regimes, the following are example of parameters and characteristics controlled in some examples.
- The number of fluid lumens penetrating the capsule. Potentially, a larger number of lumens can produce a larger number of regimes by controlling the flow from the lumens.
- The number, shape and size of each gas outlet of the gas lumens. Potentially, larger ports enable higher flows of gas.
- The shape of the outlet. Potentially, this affects air currents set up within the capsule by the flow of gas through the outlet.
- Number of gas inlets per lumen. Each lumen optionally includes more than a single inlet.
- Adjustable inlet states. Outlets are optionally opened closed, and/or partially opened, thereby controlling the outlet size and possibly also the outlet shape.
- Adjustable gas flow via to the lumen. Control *(e.g.,* by valves) of gas access to a lumen is optionally exerted for producing different gas flow regimes.
- Bursts of gas, and their duration and frequency. Optionally, the gas delivery system provides bursts of compressed gas. Optionally, duration and frequency of the bursts from each of the outlets can be controlled for producing different flow regimes.
- Positions of the outlets within the capsule. Optionally, outlet positions are controlled for producing different regimes. For example, the outlets of a first lumen are positioned at a different location than those of another lumen. Additionally or alternatively, the capsule is moveable with respect to the needles, and thereby the positions of the outlets within the capsule interior are changed. Additionally or alternatively, opening and closing different outlets changes the position of the open outlets.

With reference to *Figure 3G**,* needle **302A,** in some embodiments, includes a single gas outlet **304A,** and needle **302B** includes, for example, three gas outlets **304B, 304C** and **304D** (or any other number appropriate for the flow regime(s) to be supported). Outlet **304A** is in the shape of an elongated ellipse, outlet **304B** is in the shape of an elongated rectangle, outlet **304C** is in the shape of a square, and outlet **304D** is in the shape of a triangle. Outlet **304B** is located proximally to outlet **304C,** that in turn is located proximally to outlet **304D.** Outlet **304D** faces a different portion of capsule **300** that each of outlets **304A, 304B** and **304C.** Thus, the gas outlets can vary in shape, size, location, direction, and other physical parameters for producing various gas flow regimes within capsule **300.** It can be readily understood that other variations of these parameters also comprise embodiments of the current invention, in view of the exemplary variations just described.

With reference to *Figure 3H* and *3I*, needle **302A,** in some embodiments, is a multi-gauge needle having varying external shape and size *(i.e.,* varying external cross section). In a particular example, needle **302A** has three varying sections, proximal section **320,** middle section **322** and distal section **324.**

Optionally, sections **320, 322** and **324** are of different external cross sections. In the example set forth in *Figures 3H* and *3I*, proximal section **320** has a square shape and a diameter *(i.e.,* size) of D1; middle section **322** has a round shape and a diameter of D2 smaller than D1; and distal section **324** has an octagonal shape and a diameter of D3 smaller than D2. Thereby, in case needle **302A** pierces the capsule outlet by extending only with distal section **324** through the distal capsule wall, the capsule outlet would have an octagonal shape and a diameter of D3. In case needle **302A** pierces the capsule outlet by extending with middle section **322** through the distal capsule wall, the capsule outlet would have a round shape and a diameter of D2. In case needle **302A** pierces the capsule outlet by extending with proximal section **320** through the distal capsule wall, the capsule outlet would have a square shape and a diameter of D1. Thus, different capsule outlet having different external shape and different diameters can be produced by needle **302A,** for adapting the capsule outlet to the capsule, to the dry powder within the capsule, to the patient, and the like. It should be understood that continuous cross-section variation is also used in some embodiments of the current invention. For example, a needle is tapered, and/or has a shape which continuously varies from a first shape to a second shape.

### Methods of Outlet Preparation and Gas Delivery

### Method of Outlet Preparation and Use

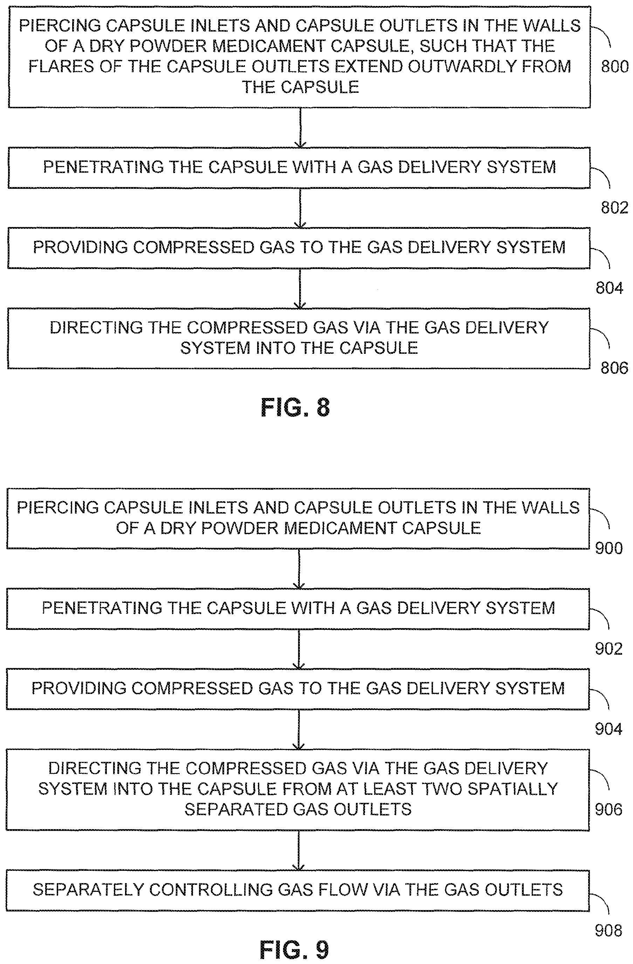

Reference is now made to *Figure 8*, which schematically illustrates a method for delivering dry powder medicament to a user and piercing outward capsule outlets, according to some examples.

At block **800,** capsule inlets and capsule outlets are pierced in the walls of a dry powder medicament capsule, such that the flares of the capsule outlets extend outwardly from the capsule. With reference to *Figures 3A* and *3B**,* needles **302** pierce capsule inlets **308** and capsule outlets **310.** It is noted that flares **312** of capsule outlets **310** extend outwardly from capsule **300.** The outwardly extending flares of the capsule outlets do not trap powder particles within the capsule.

At block **802,** the capsule is penetrated with a gas delivery system. The gas delivery system penetrates the capsule for delivering compressed gas into the capsule, for deagglomerating the dry powder within the capsule, for aerosolizing the powder, and for releasing the powder from the capsule. With reference to *Figures 3A* and *3C**,* needles **302** penetrate into capsule **300,** such that gas outlets **304** are positioned within capsule **300** for directing the compressed gas into capsule **300.** Optionally, aspects of block **800** and block **802** are combined, for example when piercing needles **302** comprise the gas delivery lumens of the gas delivery system.

At block **804,** compressed gas is provided to the gas delivery system. For example, with reference to *Figure* 2, accumulator **206** provides compressed gas to the gas delivery system *(i.e.,* to the gas lumens within needles **202).** In some examples, another pressurized gas source is used, such as a compressed gas canister.

At block **806**, the compressed gas is directed into the capsule by the gas delivery system.

### Plurality of Intra-capsule Dispersing/Deagglomerating Gas Outlets

Reference is now made to *Figure 9**,* which schematically illustrates a method for delivering dry powder medicament to a user and producing various gas flow regimes within a dry powder medicament capsule.

At block **900,** capsule inlets and capsule outlets are pierced in the walls of a dry powder medicament capsule. With reference to *Figures 3A* and *3B**,* needles **302** pierce capsule inlets **308** and capsule outlets **310.**

At block **902,** the capsule is penetrated with a gas delivery system. The gas delivery system penetrates the capsule for delivering compressed gas into the capsule, for deagglomerating the dry powder within the capsule, for aerosolizing the powder, and for releasing the powder from the capsule. With reference to *Figures 3A* and *3C**,* needles **302** penetrate into capsule **300,** such that gas outlets **304** are positioned within capsule **300** for directing the compressed gas into capsule **300.**

At block **904,** compressed gas is provided to the gas delivery system. With reference to *Figure* 2, accumulator **206** provides compressed gas to the gas delivery system *(i.e.,* to the gas lumens within needles **202).** In some embodiments, another pressurized gas source is used, such as a compressed gas canister.

At block **906,** the compressed gas is directed via the gas delivery system into the capsule, optionally from at least two spatially separated gas outlets.

At block **908,** gas flow via the gas outlets is separately controlled. For example, gas flow from the gas outlets is controlled with separately openable and closeable gas outlets, for enabling directing the compressed gas into the capsule from different gas outlets and/or combinations of gas outlets. With reference to *Figures 2* and *3D**-3F,* each of valves **204** controls the gas flow through each of needles **202** (and thereby through the gas outlets of needles **202**)**.** Thus, valves **204** optionally allow the compressed gas to be directed into capsule **210** via a first one of needles **202** *(e.g.,* needle **302A** of *Figures 3D-3F**,* as depicted in *Figure 3E*), via a second one of needles **202** *(e.g.,* needle **302B** of *Figures 3D-3F**,* as depicted in *Figure 3D**),* or via both needles *(e.g.,* needles **302A** and **302B** of *Figures 3D-3F**,* as depicted in *Figure 3F**).*

### Exemplary Inhalers

### Internal Components of an Exemplary Inhaler

Reference is now made to *Figure 2**,* which is a schematic illustration of a partial view of some of the internal components of a portable inhaler **200,** for outwardly piercing an exit aperture in a capsule, according to some exemplary embodiments of the invention.

In some embodiments, inhaler **200** includes a pair of piercing needles **202,** a pair of solenoid valves **204,** an accumulator **206** and a pump **208.**

In the example set forth in *Figure 2**,* each of needles **202** includes a gas lumen incorporated therein, and in fluid communication with accumulator **206** (or another pressurized gas source) via a respective one of valves **204.** For example, a first needle is coupled with the accumulator via a first valve, and a second needle is coupled with the accumulator via a second valve. In some embodiments, accumulator **206** is further in fluid communication with pump **208.**

In some embodiments, needles **202** are employable for piercing exit apertures **212** *(i.e.,* capsule outlets **212**) in a capsule **210** filled with a dry powder. Optionally, needles **202** are employed as passageway for gas from a gas source into capsule **210.** Needles **202** are made of materials and in a shape that enables each of needles **202** to pierce through the walls of capsule **210,** for producing apertures in capsule **210.** For example, the needles are longitudinally extended members of stainless steel. It can be understood that other shapes are possible. For example, in some embodiments, a curved needle is driven along a path which rotates a portion of its body through the capsule interior. Optionally, the inlet and outlet are thus created with any suitable relative position (not only in opposite ends, for example), according to the curvature and path of the needle.

In some embodiments, accumulator **206** comprises a compressed gas tank for storing gas compressed by pump **208.** The pressure of the compressed gas within accumulator **206** is determined by the volume of accumulator and by the amount of gas pumped into accumulator by pump **208.** Together, pump **208** and accumulator **206** form a compressed gas source (not referenced). Optionally, the compressed gas source of accumulator **206** and pump **208** can be replaced (or augmented) with other compressed gas sources. Each of the gas sources can have different quantities of gas, and can store the gas at different pressures. Each of the gas sources can include a different gas or combination of gases.

Inhaler **200**, in some examples, includes a battery (not shown) or another power source for providing power to the various components of inhaler **200,** for example, valves **204,** pump **208,** controllers, and/or sensors. O In some embodiments, inhaler **200** includes a capsule chamber (not shown) for receiving capsule **210** and/or for moving capsule **210** back and forth with respect to needles **202.**

In some embodiments, inhaler **200** includes a controller, a flow sensor, a communication port, a LED, and/or a buzzer (all not shown), optionally mounted on one or more printed circuit boards (PCB). Optionally, controller is coupled with the flow sensor, the communication port, the LED, and/or the buzzer. Additionally or alternatively, the controller is coupled with valves **204,** pump **208,** and/or the flow sensor. Where provided, the controller controls the operation of inhaler **200;** for example, according to the readings of the flow sensor.

### Capsule Preparation in an Exemplary Inhaler

In some embodiments, preparing the inhaler for use comprises the capsule chamber being brought together with needles **202** until needles **202** pierce capsule inlets in the wall of a capsule which the chamber contains. Optionally, further motion brings the needles **202** to pierce the capsule outlets at the wall of the capsule opposite to the inlets. In this manner, needles **202** pierce the capsule outlets from within the capsule itself and outwardly *(i.e.,* referred to herein as intra capsule piercing or outward piercing). Thereby, the flares of the capsule outlets *(i.e.,* ruptured capsule wall surrounding the outlets) extends outwardly from the capsule, and does not trap powder particles within the capsule during powder dispersion.

In some embodiments, the outlet piercing motion is laterally, radially, or otherwise directed to bring about a different relative position of inlets and outlets, and/or to vary the shape of an outlet. Optionally, for example, a needle comprises a blade-like portion near its tip (that is, sharpened along the length of the needle), allowing slicing as well as piercing of the capsule wall. In some embodiments, a priming operation of the inhaler causes such a needle to pivotingly slice along the capsule wall once the outlet is pierced (the pivot point being, for example, at about the point of inlet entry of the needle). Potentially, this allows wider aperture at the outlet side than was created at the inlet side of the capsule, even if needle base is larger than the needle tip. Additionally or alternatively, the needle rotates to slice; for example, in an embodiment where a blade edge is radially offset from the axis of the main needle body, optionally due to a bend in the needle. In some embodiments, the piercing is initially accomplished by a slicing motion (pivot or rotation, for example).

After piercing, in some embodiments, the capsule chamber moves away from needles **202** until the capsule outlets are clear, while maintaining the gas outlets of the gas lumens within the capsule.

Alternatively, in some embodiments, a portion of the needle forms a gas/medicament suspension outlet lumen. For example, the needle is divided into sections, a first of which has a gas inlet into the capsule chamber, and a second of which comprises an outlet aperture inside the capsule, leading to a lumen having a second aperture outside the capsule. In some embodiments, the second needle section comprises an aperture long enough to extend from the interior of the capsule to the capsule exterior, across the outlet aperture created by piercing.

With the capsule opened, and the gas outlets of the gas lumens are within the capsule, needles **202** can deliver compressed gas into the capsule for dispensing the dry powder to the patient. In the example set forth in *Figure 2* there are two needles and two valves. Alternatively, there could be another number of valves or of needles, such as one valve and one needle, one valve and three needles *(e.g.,* the valve regulates the flow of all three needles), three valves and three needles, two valves and four needles *(e.g.,* each valve regulates the flow of two of the needles), or any other combination.

In case of two or more gas outlets, inhaler **200** can generate a plurality of flow regimes of within the capsule. For example, in case of two gas outlets, inhaler **200** can deliver compressed gas into the capsule via a first outlet, via a second outlet, or via both outlets together. Thereby, three different gas flow regimes can be produced within the capsule. Furthermore, in accordance with another embodiment of the disclosed technique, the extent to which each of valves **204** is open can also be controlled. That is, each of valves **204** can have several flow levels *(e.g.,* a high, medium and low flow level). In this manner, more gas flow regimes can be produced within the capsule. Moreover, inhaler **200** can deliver the compressed gas in a series of bursts, and control the duration and frequency of the bursts by employing valves **204.** Thus, inhaler **200** can further vary the gas flow regimes (e.g., employing different frequency of bursts from each of the fluid lumens). In accordance with alternative embodiment, each of the gas lumens is coupled with a respective compressed gas source instead of being coupled to the same gas source via different valves (e.g., two gas sources for two gas lumens).

By employing different gas flow regimes, the extraction of dry powder from the capsule is potentially increased, and the amount of residual powder trapped within the capsule is reduced. Additionally or alternatively, the different gas flow regimes control (for example, increase) deagglomeration of the dry powder medicament. In some examples, this allows adapting the deagglomeration (and the release and delivery) to the specific dry powder medicament within the capsule (or to several powders, either in a single capsule or in separate capsules), to a specific patient and/or a specific treatment plan.

In some examples, the needle/capsule subassembly is formed to allow other configurations of flow besides inflow/mix/outflow and release. For example, the needle and capsule subassembly optionally comprises a circuit that allows at least some of the gas entering the capsule to exit the capsule to a route into a secondary deagglomeration chamber, and/or allows circulation of powder suspension in and out of the capsule before final release. Although adding more surfaces/chambers potentially increases residual powder losses, there is a potential advantage in allowing a longer confined exposure to energies which cause particle deagglomeration. In contrast, there is a potential reliability advantage in making the capsule wall as such the main or only outlet for particle laden gas: since the capsule is discarded after each use, there is no risk of gradual powder buildup leading to outlet plugging. As the gas inlet needle is operated under forward pressure, the risk of plugging there is low. Nevertheless, in some examples, needles are periodically replaceable, for example, as part of a disposable module. **Priming of an Exemplary Inhaler**

Reference is now made to *Figures 4A-4F**,* which are schematic illustrations of a portable active inhaler generally referenced **400,** according to some exemplary embodiments of the invention.

The preparation of the capsule described with respect to the internal components of an inhaler **200** involve various user actions for priming, examples of which are now described in relation to an inhaler **400.**

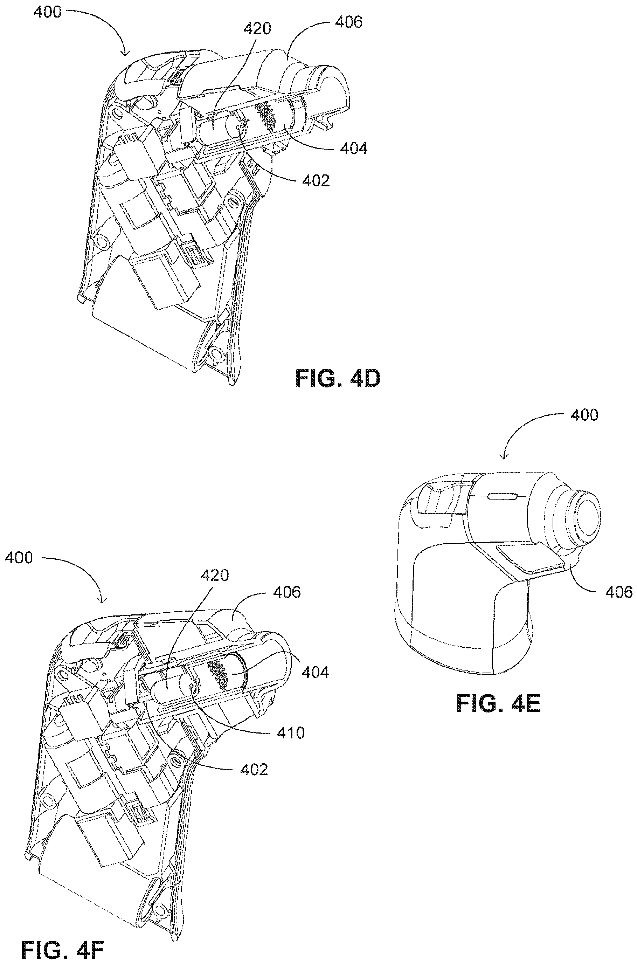

Specifically, *Figure 4A* depicts an embodiment of an inhaler **400** prior to loading a capsule **420** thereinto. *Figure 4B* depicts inhaler **400** loaded with the capsule and having with the priming knob slightly rotated. *Figure 4C* depicts a cross section view of inhaler **400** with the priming knob slightly rotated. *Figure 4D* depicts a cross section view of inhaler **400** with the priming knob almost completely rotated. *Figure 4E* depicts inhaler **400** with the priming knob fully rotated. *Figure 4F* depicts a cross section view of inhaler **400** with the priming knob fully rotated.

With reference to *Figure 4A**,* inhaler **400,** in some embodiments, includes a pair of piercing needles **402** (*Figure 4C**),* a capsule chamber **404,** a priming knob **406** and a mouthpiece **408.** Inhaler **400** further includes other components, such as all the components detailed with reference to *Figure 1**.*

In some embodiments, needles **402** serve both as piercing mechanism and as gas delivery system. Thus needles **402** are constructed such that they can withstand repeated piercing of capsules. Optionally, the needles are replaceable in the inhaler after a chosen service lifetime. Furthermore needles **402** include a gas lumen and gas outlet for delivering compressed gas from a gas source of inhaler **400** *(e.g.,* a pump and an accumulator) into a capsule **420** loaded into capsule chamber **404.** Capsule chamber **404** is movable with respect to needles **402,** such that needles **402** can pierce capsule **420** loaded into chamber **404.** It is noted that capsule **420** can be any conventional capsule *(i.e.,* capsule **420** does not have to be specifically adapted to inhaler **400),** or alternatively can be a custom made capsule. In particular, Capsule chamber **404** is adapted for receiving various types of capsules. Additionally, capsule chamber **404** can be adapted for holding more than a single capsule. Alternatively, inhaler **400** can include more than a single capsule chamber for holding more than a single capsule.

Priming knob **406** actuates capsule chamber **404** toward and away from needles **402** for piercing the loaded capsule, as would be detailed further below. Mouthpiece **408** is attached to the mouth of a patient (not shown) and provides passage out of and into inhaler **400.**

Capsule **420** containing dry powder medicament (not shown) is manually loaded into capsule chamber **404.** Prior to employing inhaler **400** for dispensing the dry powder to the patient, inhaler **400** should be loaded, primed and set up. In accordance with an embodiment of the disclosed technique, the priming and set up of inhaler **400** are performed when the user rotates priming knob **406** by 180°. In some embodiments, for example, once the user *(e.g.,* the patient or a physician) rotates priming knob **406,** a pump of inhaler **400** starts compressing air into an accumulator, serving as a compressed gas source. Additionally or alternatively, another pressure source is prepared and/or verified as appropriate.

In some embodiments, during rotation of priming knob **406,** each of piercing needles **402** pierces respective capsule inlets and outlets in capsule **420,** as described in relation to *Figures 4B-4F**.*

With reference to *Figure 4B**,* inhaler is depicted such that priming knob **406** is slightly rotated. Once the user starts rotating priming knob **406,** the priming of inhaler **400** begins. With reference to *Figure 4C**,* as can be seen in the Figure, with priming knob **406** is slightly rotated capsule chamber **404** is moved toward needles **402.** Needles **402** pierce capsule inlets in the proximal wall *(i.e.,* closest to the needles) of capsule **420.**

With reference to *Figure 4D**,* as the user continues rotating priming knob **406,** capsule chamber **404** is further moved toward needles **402.** Needles **402** pierce capsule outlets in the distal wall of capsule **420,** from within the capsule. In this manner, the flare of the capsule outlets extends outwardly from capsule **420** and does not trap powder particles within capsule **420.** Thus, the intra capsule piercing of the capsule outlets decreases the amount of wasted residual powder particles. Needles **402** can be multi-gauge so that the diameter of the capsule outlets would be smaller than that of the inlets. Moreover, the circumferential shape of needles **402** determines the shape of the capsule inlets and outlets. Additionally, different needles of needles **402** can be of different length, or different needles are separately actuated, such that the number of capsule outlets is smaller than the number of capsule inlets *(i.e.,* only some of the needles reach the distal wall and pierce a respective capsule outlet). It is to be understood that other variations of needle and/or inlet/outlet aperture position, shape, function, and/or movement as described, for example, in relation to *Figures 1**,* *2**,* and/or *3A-3I* are provided in some embodiments of the invention additionally, and/or alternatively.

With reference to *Figure 4E**,* the user has completed the 180° rotation of priming knob **406,** and inhaler **400** is primed and set up for delivering the medicament to the patient. With reference to *Figure 4F**,* after needles **402** pierced outlets **410** in the distal wall of capsule **420,** as priming knob **406** completes its 180° turn, chamber **404** is moved away from needles **402.** Thus, outlets **410** of capsule **420** are free and allow extraction of the dry powder therethrough. Additionally, the gas outlets of the gas lumens of needles **402** are positioned within capsule **420.** In some embodiments, another final motion is performed to bring about the intended final position of the needles and their apertures relative to the capsule, for example, any position as described hereinabove. In this manner, compressed gas passing through needles **402** is evacuated within capsule **420** and is employed for deagglomerating, aerosolizing, and/or releasing the dry powder within capsule **420.** Needles **402** are coupled with the accumulator (or other pressure source) via respective valves, and thus several gas flow regimes can be produced within capsule **420.**

In some examples, an at least one switch is activated when the rotation is completed, turning on the electric circuit which places the inhaler **400** into a stand-by mode. Optionally, an indication is provided to the user that the device is ready to deliver the drug, for example, by illuminating a signaling LED, and/or providing another visible, audible, and/or tactile signal.

### Gas Delivery in an Exemplary Inhaler

Returning now to *Figure* 2, the operation of inhaler **200** to deliver pressurized gas to a prepared and primed dry powder capsule is detailed hereinbelow.

In some embodiments, a user inserts capsule **210** into the capsule chamber and primes inhaler **200** by rotating a priming knob, for example as described with reference to *Figures 4A-4F* hereinbelow. Optionally, priming of inhaler **200** involves pump **208** pumping air into accumulator **206,** and/or other preparation of the pressurized gas source. According to an aspect of the invention, priming of inhaler **200** further involves moving the capsule chamber together with the needles for piercing capsule inlets and outlets in the walls of the capsule, and/or retracting the capsule chamber for positioning the gas outlets *(i.e.,* of the gas lumens within the needles) within the capsule.

In some examples, pump **208** inflates accumulator **206** to an appropriate pressure, optionally the highest pressure that pump **208** is capable of (*e.g*., pressure of 1 bar, 5 bars, 10 bars or another greater, lesser, or intermediate pressure). By employing an accumulator to store the compressed gas, the inhaler can deliver larger volumes of compressed gas at the desired pressure, than the volumes that are produced in real time by the pump itself.

In some examples, the flow sensor detects the inhalation flow rate of the patient. When the controller determines that a desired flow rate threshold is exceeded (or that another release condition is reached), inhaler **200** disperses the powder content out of the capsule, while breaking the powder particles to a powder fineness appropriate to reaching the target site. Optionally, the controller compares the patient performance to the required performance (*e.g*., in terms of flow rate or inhaled volume) and employs the buzzer and/or the LED light to correct and guide the patient in real time. Data are optionally stored in an internal memory, from which the physician can optionally download the data via a communication port. Optionally, the physician can receive any information about the way the patient used the inhaler in real time, and/or throughout the last minutes, days, weeks, months and/or years.

Once the desired inhalation threshold is achieved, pump **208** optionally starts working again, while valves **204** (for example, solenoid valves) are opened and closed to produce preprogrammed and/or dynamically determined gas flow regime(s) within capsule **210** (*e.g.,* pulses of air flow in the capsule or continues flow from both or one of the needles). Thus, inhaler **200** includes an active delivery system that is optionally operated in a closed-loop control. It is noted that the dry powder medicament can be selectively delivered to the patient only during the inhalation phase, for example, in a single inhalation phase or in a plurality of inhalation phases.

### Other Aspects of the Active Inhaler and its Use

### Inhaler Mouthpieces

Returning now to *Figure 1*: to operate the inhaler, the inhaler mouthpiece **126** is placed to the mouth of the patient. Mouthpiece **126** is an adapter for conveniently attaching airway **104** to the mouth of the patient. The patient inhales the delivered dry powder medicament through mouthpiece **126.** Alternatively, mouthpiece **126** can be replaced by a facemask which is attached to the face of the patient. Further alternatively, mouthpiece **126** is replaced with a nasal adapter for coupling airway **104** to the nose of the patient.

The facemask (or the nasal adapter) is optionally employed for patients that require drug delivery over several breathing cycles, such as children, elderly people and people with respiratory problems. Additionally, the facemask can be used for patients that cannot hold the mouthpiece to their mouth, such as handicapped patients, unconscious patients, and the like. Furthermore, the facemask can be employed for patients, to whom it is difficult to explain how to employ the inhaler, such as people with mental problems. Additionally or alternatively, a potential advantage of dividing administration over several inhalation cycles is to prevent administering enough powder in one to trigger a cough reflex. This can be in order to make individual deliveries smaller, and/or to allow a larger powder load (likely to trigger a reflex cough, for example, if delivered all at once) to be divided among multiple inhalation cycles.

### Active Gas Sources

In some examples, pump **116** and accumulator **118** form together a compressed gas source (not referenced). In particular, accumulator **118** is a chamber for storing compressed gas, compressed by pump **116.** Optionally, accumulator **118** is coupled with a pressure sensor for determining the pressure of the compressed gas. In the example set forth in *Figure 1*, pump **116** is an automatic pump consuming electrical power provided by a power source (*e.g.,* a battery). Alternatively, pump **116** can be manual compressing device operated by the user. In this case, controller **108** can provide guidance to the user respective of the desired operation of the manual pump. In accordance with an alternative embodiment, pump **116** and accumulator **118** are replaced by another compressed gas source, or are augmented with additional compressed gas sources. For example, the inhaler includes a first gas source including compressed air, and a second gas source of Heliox.

The compressed gas is directed into a capsule (not shown) of medicament dry powder loaded into capsule chamber **124** (or into more than a single capsule loaded into chamber **124** or into several such chambers). The dry powder includes a medicament or combination of medicaments, and may further include other substances such as inert carriers (*e.g*., lactose, glucose). The compressed gas is employed for deagglomerating, aerosolizing, mixing or simply for releasing the dry powder from the capsule.

The compressed gas can be room air compressed into the accumulator. Alternatively, the compressed gas can include one of more of the following: oxygen, oxygen and room air, helium and oxygen (*i.e.,* Heliox), and/or an anesthetic gas. Furthermore, the compressed gas can contain therapeutic ingredients (medication). Optionally, the compressed gas contains ingredients that condition the dry powder drug deagglomeration and/or the dispersion process. Optionally, the inhaler includes more than a single gas source. For example, the inhaler includes several gas sources, each containing a different gas that can be separately delivered into the capsule. Alternatively, a single gas source includes a mixture of different gases. It is noted that the inhaler can include more than a single gas source for the same gas, for example, a first gas source at which that gas is stored at a first pressure and a second gas source at which that gas is stored at a second pressure.

### Adaptive Airway Restriction

In some embodiments of the current invention, valve **102** can be controlled by controller **108** (or by a separate controller) or manually controlled, such that it actively controls the inhaler restriction and can block the flow intermittently. As mentioned above, the airway restriction affects the drug deposition. Therefore, actively adapting the restriction can improve drug delivery. The restriction of airway **104** can thus be adapted, for example, to different powders, different patients, adapted according to the sensed breathing of the patient, and the like. In some embodiments, a fixed restriction (a region of reduced cross-section, for example) is provided along the airway **104.**

In some examples, inlet valve **102** is controlled in a closed loop manner according to the inspiratory flow of the user. Thus, the restriction is automatically adapted to the breathing of the patient. Optionally, a restriction (before or after the capsule in the airway) is adjusted to control a pressure differential developed across the capsule by the inhalation of the patient (for example, in a device where gas flow through a needle is generated by pressure differentials due to patient respiration). Alternatively, inlet valve **102** is omitted from inhaler **100.** Flow sensor **106** monitors the flow of fluids through airway **104.** Flow sensor **106** can be implemented for example, by a pressure sensor or hot wire sensor, and the like. Flow sensor **106** provides the flow readings to controller **108** that determines accordingly various breath parameters of the patient. For example, controller **108** can determine inspiratory or expiratory flow rate, volume, phase, breath patterns, and the like.

### Closed Loop Control and User Feedback

In some examples, inhaler **100, 200,** and/or **400** provides feedback to the patient and the physician for monitoring the treatment, in real time. For example, the inhaler indicates to the user when it is ready for action, how to improve breathing for optimal drug delivery, and/or what amount of medicament was delivered. The feedback to the patient is performed by feedback interface **112,** which can include visual, audio and haptic interfaces (e.g. LED lights, speaker, vibrator, and similar output interfaces). Optionally, the inhaler informs the physician about the breathing pattern of the patient and the patient compliance. The inhaler can further log additional data to be presented to the patient and the physician; for example: the time of each drug delivery, the duration of the delivery, the amount that was delivered, the breathing of the patient before, during and following delivery, the type of medicament delivered, and/or the size of the delivered particles (as determined by the deagglomeration of the powder).

In some examples, flow sensor **106** provides flow readings to controller **108.** Thereby, flow sensor **106** enables controller **108** to coordinate the release of the dry powder with the breathing of the patient, to provide feedback to the patient, and the like. For example, controller **108** calculates the inspiratory flow rate of the patient according to the flow readings, and provides feedback to the patient for guiding how to optimize the dispensing of the medicament dry powder by modifying her breathing. In accordance with an additional example, inhaler **100** starts drug delivery when the inspiratory flow rate exceeds a threshold, and stops delivery in case the inspiratory flow rate decreases to below the threshold.

In some examples, inhaler **100, 200** and/or **400** (*i.e.,* flow sensor **106** and controller **108**) measures the expiratory flow rate for determining the lung capacity of the patient. Thereby, the drug delivery can be adapted to the anatomy of the patient. For example, the lung capacity of child (*i.e.,* and the inspiratory volume) is smaller than that of an adult, and inhaler **100** can take that into consideration when coordinating active delivery of the dry powder medicament. Additionally, the expiratory flow rate of the patient can further be measured, for example, for verifying that the patient has emptied her lungs and would be ready to inhale the dispersed medicament during the following breathing cycle.

Controller **108** is, for example, a microcontroller or any other processing device that can gather data from the sensors and components of inhaler **100,** as well from external sources, and accordingly control the operation of the various components of inhaler **100.** For example, controller **108** can be implemented by a programmable chip, an application specific integrated circuit, a nearby processor configured to perform the functions describe herein (*e.g.,* a desktop computer, PDA and/or cellphone), a remote processor, such as a central server, and the like. Controller can further include a memory portion, storing thereon, for example, software instructions, parameters for release conditions, range settings, look-up tables, calibration measurements, and/or saved patient data.

Data logger 110 is a module that handles all data logging operations of inhaler **100,** such as breathing patterns data, dry powder dispensed quantities, dates and time, and the like. Data logger **110** enables a user, such as the patient and/or physician, to analyze treatment data in order to improve clinical outcomes.

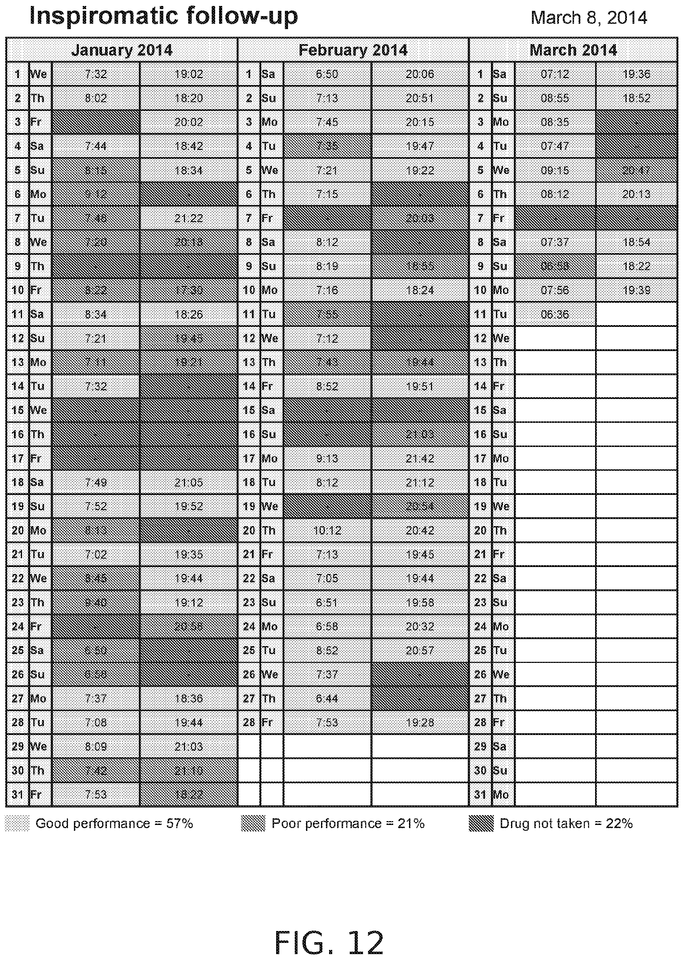

Reference is now made to *Figure 12*, which shows an exemplary logged data summary sheet for an inhaler, according to some examples. In some examples, a report of logged data comprises indications of when a medicament was taken and/or not taken (for example, day of week, day of month, month and/or time of day). Along with the time indication, an indication of good performance, poor performance, and/or drug not taken is optionally provided. Potentially, such a data sheet allows monitoring of patient performance, and if performance and/or compliance falls below an established norm, corrective action can potentially be taken. For ease of reference, performance is optionally summarized as percent good, percent poor, and/or percent of scheduled administrations not taken.

### Auxiliary Sensing, Monitoring, and Control

In some examples, a gas sensor is placed in fluid connection with mouthpiece **126** or with airway **104,** and is further coupled with controller **108.** The gas sensor detects levels of exhaled compounds (e.g. levels of carbonyls or glucose), stores the exhaled compounds data on data logger **110** and informs the user via feedback interface **112.** Moreover, the gas sensor can optionally be used to monitor the therapeutic effect of the delivered drug and control the present delivery accordingly in a closed loop.

Additionally or alternatively, inhaler **100** is coupled with other sensors (either incorporated therein or external thereto) to monitor other aspects of the drug delivery process, and for modifying the drug delivery process accordingly. For example, the controller receives data from a sensor monitoring the blood pressure of the patient, monitors blood constituents, or other physiologic parameters of the patient. For instance, the controller is coupled with an external sensor that measures the oxygen levels in the blood of the patient. According to the measured oxygen levels the controller determines whether enough medicament powder was delivered.

In some examples, the amount of dry powder released from the capsule is determined by the flow of compressed gas into the capsule. Optionally, the amount of released powder is controlled by valve **102** that can be timed to shut by controller **108** according to a desired delivery amount, according to a measurement of the flowing powder, and the like.

Optionally, one or more sensors are used for detecting one or more of a variety of release conditions, such as flow rate sensor, heart rate sensor, blood analyzing sensors, vacuum sensors, and the like. As mentioned above, a flow sensor acquires flow readings of the inspiratory (or expiratory) flow rate of the patient. Examples of flow sensors include a laminar flow meter, a thermal flow meter, a coriolis flow meter, an ultrasonic flow meter and/or a variable area flow meter. Moreover, the inhaler is optionally coupled with external sensors via the communication port. For example, the inhaler receives the readings of a glucometer and a blood pressure sensor.

### Inhaler User Feedback for Training

In some aspects, a feedback interface **112** is provided as a user interface for providing feedback to the user to improve the utility of inhaler **100, 200, 400.** Optionally, feedback interface **112** provides feedback to the user with respect to the breathing parameters and the medicament delivery parameters, in real time. For example, feedback interface **112** indicates to the user that an inhalation flow rate is too low and should be increased. In accordance with another example, when a threshold inspiratory volume is exceeded (*i.e.,* indicating that a sufficient amount of medicament powder was inhaled), feedback interface **112** signals to the patient that the prescribed amount of powder was delivered, or that the delivered drug reached the target site. Thus, the patient is reassured that the drug was delivered. Patient reassurance is potentially important for the ease of mind of the patient, as is compliance with the prescribed treatment. Furthermore, patient reassurance can induce a desired placebo effect in addition to the chemical and biological effects of the delivered drug.

Optionally, feedback interface **112** is used for training purposes. In this case, the inhaler is optionally used with no drug loaded, or loaded with a drug capsule containing placebo. The patient attaches the inhaler mouthpiece (or facemask) to her mouth, and inhales. Feedback interface **112** indicates to the patient how to modify her breathing for optimizing drug delivery. Optionally, the interface is designed to monitor exhaling, and/or exhaling and inhaling together, for example to train a user how to clear the lungs of air before use of the inhaler - even for examples where usual operation is triggered based on inhalation alone.

Once the patient knows how to optimize drug delivery via the inhaler, the patient can load a dry powder medicament capsule into the inhaler and begin treatment. Thereby, drug delivery is potentially increased in reliability. Optionally, the feedback interface provides feedback to the physician for allowing making treatment decisions accordingly (*e.g*., changing the dose of the medicament or instructing the patient about recommended breathing patterns). In some aspects, inhaler **100** can be employed as a diagnostic device, such as, a spirometer.

In some aspects, communication port **114** comprises a communication link allowing inhaler **100** to connect to external devices, such as data sources, processing devices, medical tools (*e.g*., diagnostic or treatment tools), and the like. Communication port **114** can be implemented by various communication links, such as wired communication links (*e.g.,* USB port), wireless communication links (*e.g.,* optical, radio frequency, sonic, ultrasonic, cellular), or a combination of more than a single communication link.

### Inhaler with Replaceable Head

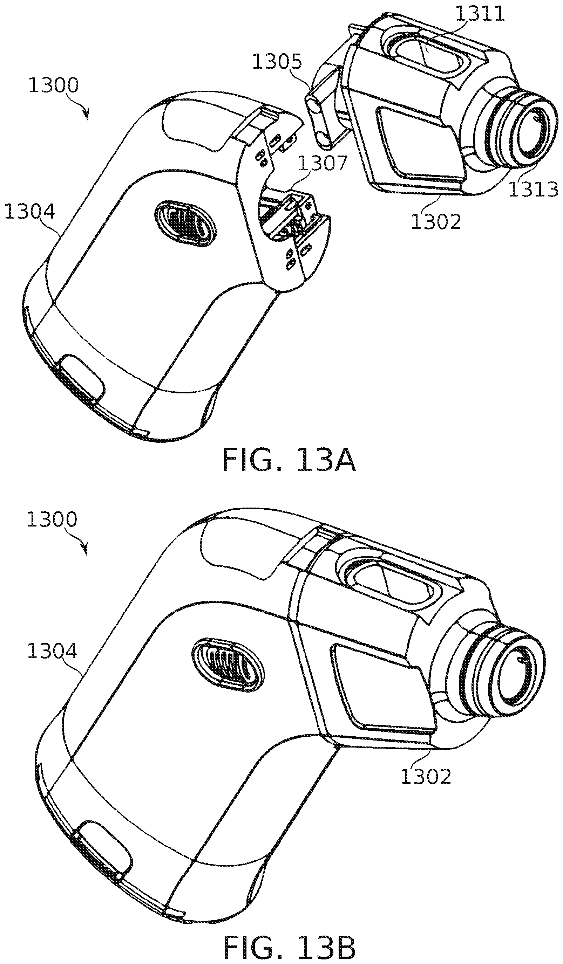

Reference is now made to *Figures 13A-13C*, which schematically illustrate an inhaler **1300** with replaceable head **1302,** according to some exemplary embodiments of the invention.

In some embodiments, an inhaler **1300** comprises a base **1304** and a replaceable head section **1302.** The base **1304** and head **1302** are mated through connector regions **1307, 1305.**

In some embodiments, head **1302** comprises the capsule chamber **1311,** mouthpiece **1313** (alternatively, a facemask, nasal adapter, and/or interface for connecting to a facemask/adapter), and needles (not shown) which puncture and/or deliver gas to the capsule. In some embodiments,, the main body of the head **1302** is rotatable with respect to the connector **1305,** for example as described in relation to *Figures 4A-4F**;* optionally, the rotation is used for preparatory tasks such as loading the capsule and priming the inhaler for medicament delivery. In some embodiments, one or more motions (for example, an extra rotation, a catch release, a pulling apart, and/or another motion) are used to enable removing the head, with complementary motions to replace it with a new one.

In some embodiments, the adapter connection between regions **1305** and **1307** comprises connections which provide delivery of air, and/or electrical power for functions including, for example, actuating the needle, sensing, and/or illuminating notification lamps. Optionally, all movement functions of the head **1302** (for example, capsule insertion and/or needle puncturing) are performed by mechanical operations such as relative rotation of the head body relative to the connector region **1305.** Optionally, no electrical power connection to the head is required, and functions such as indications and sensing are contained entirely within the base section **1304.**

In some embodiments, the base section **1304** comprises a compressed gas source (for example, a pump and accumulator), a controller, and/or a power source such as a battery. Optionally, sensors and/or notification means (LEDs, sound generators) are also housed in the base section. Optionally, all sensors and/or notification means are housed in the base section.

In some embodiments of the invention, head **1302** is a disposable element, used, for example, with a predetermined number of capsules before being discarded. For example, the head is used with 10 capsules, 25 capsules, 40 capsules, 60 capsules, 100 capsules, or another larger, smaller or intermediate number of capsules before being discarded. This provides a potential advantage by providing for regular replacement of inhaler portions liable to becoming contaminated with old medicament powder over time. Potentially, this reduces the chances of adverse occurrences such as clogging, needle dulling, inadvertent delivery of a too-large dose accumulated powder which suddenly dislodges, inadvertent delivery of an API breakdown product, and/or another undesired occurrence which could potentially result from wear, ageing, and/or contamination of the portions of the inhaler directly in contact with medicament during release. Optionally, each head is provided with the full number of capsules to be used together with it as a complete kit. In some embodiments, the head comprises storage for one, some (for example, 5-10 or more), up to all capsules which are provided for use with the head.

While the head is optionally replaced more often than might be economically practical for the full replacement one-piece inhaler design, another potential advantage of a two-piece design is that the base section **1304** of the device is optionally kept in service for longer than would be practical for a single-piece design. For example, the base section is kept in service for use during a service lifetime of 10 heads, 20 heads, 50 heads, 100 heads, 250 heads, or another larger, smaller, or intermediate number of heads. A potential advantage of a longer service life is reduced cost for providing the system, insofar as more expensive system components such as power, gas supply, controller, sensor, and/or indicators are optionally provided within the base section **1304.**

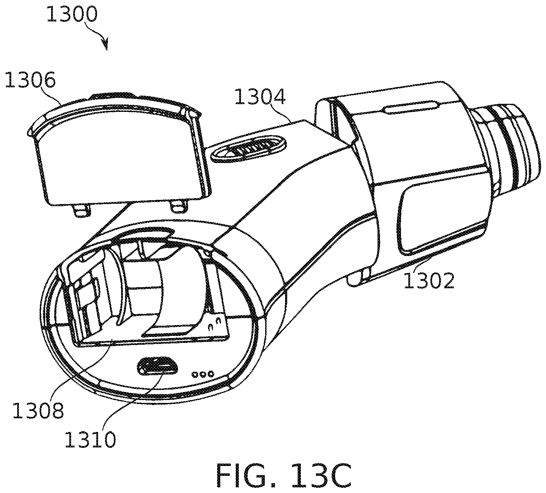

In some examples, there is provision made for battery replacement and/or battery recharging. *Figure 13C* shows an open battery compartment **1308,** accessed through a battery service door **1306.** Optionally, the battery held by compartment **1308** is a standard battery, for example, an off-the-shelf CR123A battery. Optionally, the battery is rechargeable, for example by connection through a charging connection **1310** (which is optionally also a data connection, for example as found in USB interfaces). In some examples, a reserve power battery is provided in addition to the main battery. This provides a potential advantage for functions such as preserving device memory and time keeping during replacement of the main battery.

### Inhaler Cap

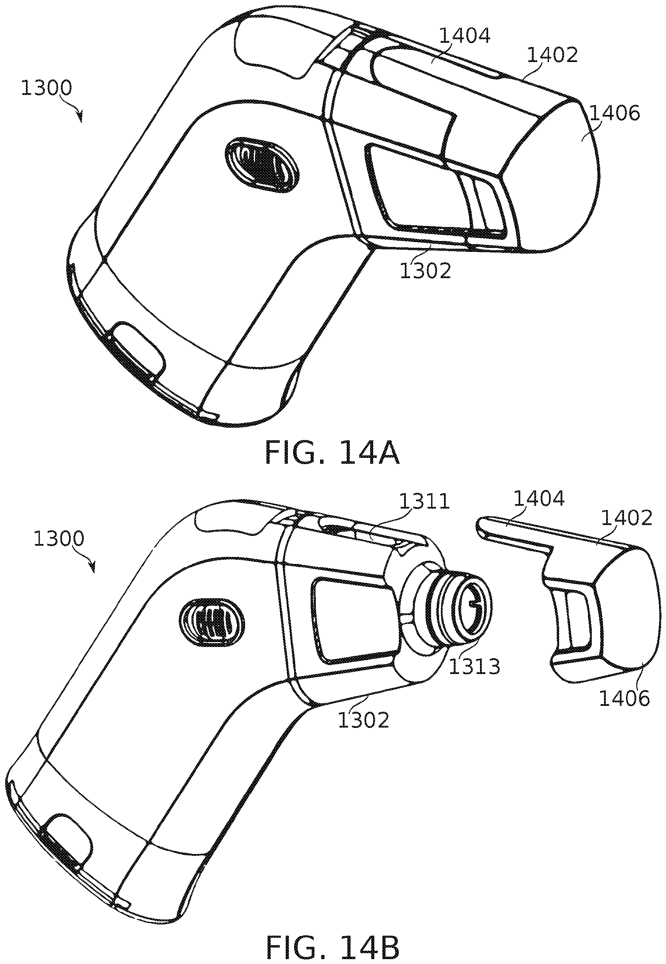

Reference is now made to *Figures 14A-14B*, which show a cap **1402** for an inhaler **1300** in place and after removal, respectively, according to some examples. In some examples, a cap **1402** is provided for use with the inhaler **1300** (optionally, provided together with an inhaler head **1302).** In some examples, cap **1402** interferes with the motion of head **1302** (or head subassembly), to prevent accidental priming while the cap is in place. For example a tongue **1404** of the cap protrudes to where it prevents one or more of the motions of priming. Optionally, cap **1402** also protects the inhaler/patient interface (to keep it clean and/or dry), for example, by covering mouthpiece **1313** with a protective shield region **1406.** In some embodiments, tongue **1404** or another surface of the cap **1402** acts to enclose the capsule chamber **1311.** Optionally, this allows a capsule to be stored in the capsule chamber **1311** without risk of accidentally priming it, so long as the cap **1402** remains in place.

### Examples of Operation Details

### Synchronization to Respiration

Reference is now made to *Figure 5*, which is a schematic illustration of breathing rate curve **500,** according to some exemplary embodiments of the invention.

Breathing rate curve **500** (*i.e.,* or flow rate curve **500**) depicts the flow rate of the patient as calculated according to a flow sensor of an inhaler (*e.g*., inhaler **100** of *Figure 1*), versus the time, during a single breathing cycle of the patient. The breathing cycle includes an inspiratory phase and an expiratory phase.

A flow 'threshold', represents the minimal flow rate of the patient at which drug is delivered. The threshold is determined according to the type of dry powder delivered, according to the desired target area for delivery of the powder, and according to other parameters. The flow threshold, as well as other delivery parameters, can be pre-set in the device software (*e.g*., by the inhaler manufacturer or by the manufacturer of the dry powder medicament). Additionally or alternatively, the flow threshold and other delivery parameters are set by the physician, by the patient, or by a caregiver.

A time 'delay' represents the duration of waiting period (*i.e.,* a pause) of the inhaler device from the beginning of a breathing cycle (*i.e.,* of the inspiratory phase of the breathing cycle) before delivering the dry powder. An 'inert' part of the inspiratory phase represents the final portion of the inspiratory phase, at which inhaled air does not reach the lungs and only fills the upper respiratory system (*e.g.,* the large airways and the pharynx).

The "powder release" period represents the time of delivery of the dry powder medicament within the capsule toward the patient. Specifically, the width of the "powder release" represents the duration of the drug delivery burst (or cluster of bursts, if gas delivery is so-configured). The dry powder can be fully released during one inhalation, or released over several breathing cycles (*i.e.,* several inhalations). Additionally, the drug can be delivered in a continuous flow, or in a cluster of bursts. The delivery bursts can occur during a single inhalation or over several inhalations.

### Single Inhalation Operation

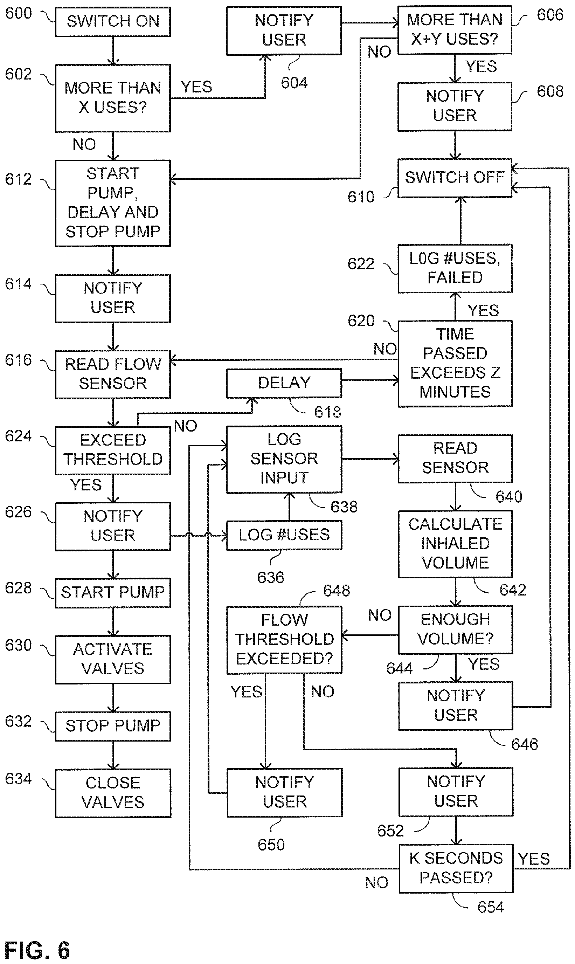

Reference is now made to *Figure 6**,* which is a schematic illustration of an exemplary method for operating an inhaler for delivering dry powder medicament during a single inhalation of the patient, according to some examples. Typically, but not exclusively, this flow of operations is used with an inhaler operated together with a mouthpiece.

At block **600,** the inhaler device is switched on by a user. It is noted that the inhaler device is switched on (or at least, is only ready for use) after being preloaded and primed with a dry powder capsule, for example as described in relation to *Figures 4A-4F**,* including any operations of piercing required. In some embodiments, the system controller detects the loaded/primed state of the inhaler, and provides indications of ready/not ready state accordingly.

In some examples, the inhaler device, or an operational portion thereof, is disposed of after a predetermined number of uses. For example, in case the inhaler should be discarded after 700 uses, the inhaler can notify the user when it exceeds 650 uses, to enable the user to prepare in advance for discarding and replacing the inhaler device. Once the inhaler device exceeds 700 uses, it can be locked from further use. Blocks **602-610** as detailed hereinbelow describe the determination of the number of uses, and the notifications to the user.

At block **602,** it is determined whether the inhaler has been used more than X times (*e.g*., more than 100, 200, 700 or 1500 times, or another larger, smaller, or intermediate number of uses). The number of uses of the inhaler is recorded by a data logger module of the inhaler. In case the number of uses exceeds a pre-determined number of uses, the method continues to block **604;** otherwise the method continues to block **612.**

At block **604**, the user is notified that the number of uses of the inhaler exceeds a threshold and that the inhaler should soon be replaced. The user may further be notified the remaining number of times the inhaler can be used before the inhaler should be replaced. For example, in case the inhaler should not be used more than 700 times, the user can be notified once the number of uses exceeds 650 so that she can prepare in advance for replacing the inhaler. Thus, a threshold number of uses for indicating to the user that the inhaler should be replaced soon is 650.

At block **606,** it is determined whether the inhaler has been used more than X+Y times (*e.g*., more than 120, 220, 750 or 1550 times). X+Y represent the number of uses of the inhaler recommended by the manufacturer of the inhaler. At **608,** after the number of allowed uses is exceeded, the user is notified that the inhaler should be replaced. For example, in case the inhaler should be replaced after 700 times, first for each use over 650 uses the user is notified that the inhaler should be replaced soon, and when reaching 700 uses the user is notified that the inhaler should be replaced.

At block **610,** the inhaler is switched off such that it cannot be further used. For example, in case the number of uses of the inhaler exceeds the recommended number of uses determined by the manufacturer, the inhaler can deactivate itself and prevent the user from further employing the inhaler. Thus, the safety of the inhaler is increased by enforcing the allowed uses threshold.

At block **612,** in case the number of uses is below the threshold, the pump is activated for compressing gas into the accumulator of the inhaler; thereby the accumulator becomes a compressed gas source. At block **614**, the user is notified that the inhaler is ready for use. The user is notified by an output interface of the inhaler, such as a buzzer, a LED, a screen, other visual and or audio output interfaces, and the like.

At block **616,** flow sensor readings are received respective of breathing characteristics of the patient. At **624,** it is determined whether a predetermined threshold for the breathing characteristics is exceeded. For example, it is determined whether the threshold inhalation flow rate or volume was exceeded (*e.g.* 10 liters per minute). In case the detected breath characteristic exceeds the predetermined threshold, the method continues in block **626,** otherwise the method continues in block **618**.

At block **618,** the inhaler delays its operation until the predetermined threshold for the breathing characteristics (blocks **616** and **624**) is exceeded. At block **620,** it is determined whether a predetermined period has been exceeded. In case the predetermined time period *(e.g.,* 5 minutes) is exceeded, the method continues in block **622.** Block **620** is directed at preventing the inhaler from being kept turned on without delivering the medicament, thereby draining the battery. In case the patient has decided not to use the inhaler, or in case the patient cannot reach the breathing parameter threshold for a predetermined period, the inhaler logs the failed attempts and turns off (blocks **622** and **610**). During the delay of block **620,** the flow sensor keeps on acquiring breathing readings (block **616**) and in case the threshold is exceeded (block **624**), the method continues to **626.** At block **622,** the failure to achieve the breath characteristic threshold is recorded along with metadata, such as the date and time, the use number of the inhaler, at which patient failed to reach the breathing threshold, and the like. Thereafter, at block **610,** the inhaler switches off.

At block **626,** the user is notified that the breathing threshold was exceeded and that drug delivery can commence. Thereby, patient breathing (*e.g*., inhalation flow rate) is employed for triggering drug delivery, that is, employed as a release condition. At blocks **628, 630, 632** and **634,** the pump is activated for compressing gas into the accumulator, and the valves are opened and closed for delivering the compressed gas into the capsule for deagglomerating, aerosolizing or releasing the dry powder from the capsule.

Additionally, after notifying the user that the breathing threshold has been exceeded (block **626**), the method also follows block **636.** At block **636,** the number of uses, the data and the time of the current use of the inhaler are recorded (*i.e.,* logged). At **638,** the flow sensor readings are logged. At block **640,** flow sensor additional readings are gathered, and at block **642** the inhaled volume is accordingly calculated. Alternatively, other breathing parameters can be calculated, such as exhaled volume.

At block **644,** it is determined whether the inhaled volume exceeds a predetermined threshold. In case the inhaled volume exceeds the respective threshold, the method continues in block **646,** otherwise the method continues in block **648.** At block **646,** the user is notified that the inhaled volume was sufficient and that the drug delivery was successfully completed. Thereafter, at **610,** the inhaler switches off.

At block **648,** in case the inhalation volume was not exceeded, it is determined whether the breath flow threshold was exceeded. If the breath flow threshold was exceeded the method follows block **650,** otherwise the method follows block **652.** At block **650,** the user is notified that the inhalation volume threshold was not exceeded and that the breath flow threshold was exceeded. Thereafter, the method returns to block **638.**

At block **652,** the user is notified that the inhalation volume has not reached the threshold, and that the breath flow threshold was also not exceeded. At block **654,** as long as the time that passed since performance of block **652** does not exceed a short predetermined time period (*e.g.,* 20 seconds), the method returns to block **638.** Otherwise, in case the time that passed since block **652** exceeds the predetermined short time period, the moves to block **610** and the inhaler switches off. Moreover, the inhaler can notify the user before switching off, and can further log inhaler data before switching off.

### Plurality of Inhalations (Facemask Operation)

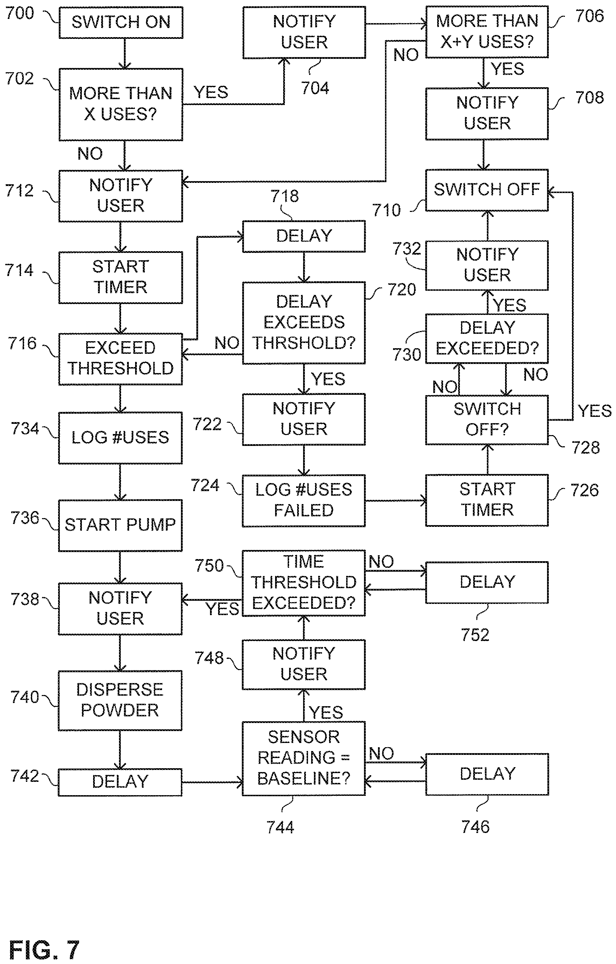

Reference is now made to *Figure 7*, which is a schematic illustration of an exemplary method for operating an inhaler for delivering dry powder medicament in a prolonged manner over consecutive breathing cycles of the patient, according to some examples.

Blocks **700-710** of the method of *Figure 7* are substantially similar to blocks **600-610** of the method of *Figure 6* described hereinabove.

At block **712,** in case the number of past uses of the inhaler is below the threshold (block **702**), the user is notified that the inhaler is ready for use. The user is notified by an output interface of the inhaler, such as a buzzer, a LED, a screen, other visual and or audio output interfaces, and the like.

At block **714,** a timer is started. At block **716,** in some embodiments, a flow sensor reading is acquired and it is determined whether a breathing parameter threshold was exceeded. For example, the inhaler determines whether the inspiratory flow rate of the patient exceeds a threshold. In case the breathing parameter threshold of 716 is exceeded the method continues to block **734,** otherwise the method follows block **718.**

At block **718,** inhaler delays its operation for a short time period (*e.g.,* 100 ms). At block **720,** in some embodiments, in case the timer (block **714**) exceeds a determined time period (*e.g.,* 10 minutes), the method continues to block **622,** otherwise the method returns to block **716** and checks whether the breathing parameter threshold was exceeded during the short delay.

At block **722,** in case the timer exceeded the determined time period, the user is notified that the time period was exceeded and that the breathing parameter threshold was not exceeded during that time period. At **724,** the number of uses, the date, the time and the failed delivery are logged via the data logger of the inhaler. At block **726,** in some embodiments, the timer is started. At block **728** it is determined whether to turn off the inhaler (return to block **710**) or to wait and follow block **730.** At block **730,** the inhaler delays its operation for a short time period *(e.g.,* 1 second) and returns to block **728.**

At block **734,** the number of uses of the inhaler, and the current date and time, are logged via a data logger of the inhaler. At block **736,** a pump of the inhaler is activated. Alternatively, in case the compressed gas source of the inhaler is not composed of a pump and an accumulator, the compressed gas source is primed (*i.e.,* in case it requires priming). At block **738,** the user is notified that drug delivery is about to commence.

At block **740,** the inhaler directs the compressed gas into the capsule for deagglomerating, dispersing and releasing the dry powder medicament. For example, inhaler **200** of *Figure 2* herein above, directs the compressed gas into the capsule by opening valves **204** together for 70 ms and then closing both valves for 30 ms (*i.e.,* thereby producing a first gas flow regime within the capsule). Thereafter, only a first one of valves **204** is opened for 70 ms and then both valves are closed for 30 ms (*i.e.,* thereby producing a second gas flow regime within the capsule). Lastly, the second one of valves **204** is opened for 70 ms and then both valves are closed for 30 ms (*i.e.,* thereby producing a third gas flow regime within the capsule).

At block **742,** inhaler delays its operation for a short time period (*e.g.,* 300 ms). At **744,** a flow sensor reading is acquired, and it is determined whether the sensor reading exceeds a predetermined baseline. In case the baseline is exceeded, the method continues to block **748,** otherwise the method follows block **746.** At block **746,** the inhaler delays its operation for a short time period (*e.g.,* 50 ms), and then the method returns to block **744.**

At block **748,** the user is notified that the sensor baseline reading was exceeded. At block **750,** it is determined whether the breathing parameter threshold was exceeded. In case the threshold was exceeded, the method returns to block **738** for another round of drug dispersion (block **740**), otherwise the method follows block **752.** At block **752,** the inhaler delays its operation for a short time period (*e.g.,* 50 ms), and then the method returns to block **750.** It is noted that at **750,** the inhaler already dispersed the powder at least once (during an inhalation of the patient), and is ready to repeat drug dispersion. In this manner, the method of *Figure 7* is employed for controlling the inhaler for delivering the dry powder medicament in a prolonged manner, over consecutive breathing cycles of the patient.

### Alternative Multi-Inhalation Operation

Reference is now made to *Figures 10A-10C**,* which together comprise a schematic illustration of an exemplary method for operating an inhaler with a disposable head for delivering dry powder medicament during a plurality of inhalations of a patient, according to some examples. Typically, but not exclusively, this flow of operations is used with an inhaler operated together with a mask or nasal adapter. Mask and/or adapter use is typically with patients for whom controlled deep inhalation is difficult to obtain for some reason, so that acquisition over several breath cycles is likely to be required.

This flow of operations is for use with an inhaler having a disposable head section. A general difference from a fully disposable inhaler design is that the primary limitation on extended use is battery charge, rather than number of uses. *Figures 10A-**10C* also provide exemplary detail relating to timing of valve operation during medicament dispersal, and of other timings related to error conditions, timeouts, and/or polling cycles.

Although the flow of operations is described in connection with specific timings for various operations (for example 30 msec, 50 msec, 100 msec, 5 sec, 1 min, 3 min, 5 min, 10 min), it is to be understood that these timings should be taken as exemplary and not limiting. Although the flow of operations is described in connection with specific indications (for example blue LED, green LED, red LED, beep, blink), it is to be understood that these indications should be taken as exemplary and not limiting. It should be understood more generally that this flowchart of operations is only one particular example of operations for medicament delivery, and that other flows of operations comprising use of the invention, but having more, fewer, and/or different blocks also represent examples. Thresholds for activation (described, for example, in relation to a 10 1/minute inspiration rate) should also be understood to be variable to other settings: for example, 1 l/min, 5 l/min, 15 1/min, or another greater, lesser, or intermediate inspiration rate. The rate is generally chosen, for example, to have sufficient magnitude to effectively guarantee that drug will be taken into the target tissue and/or to have sufficient magnitude that it is unlikely that the flow will reverse during a drug delivery epoch.

The flowchart begins with *Figure 10A**.* At block **1002,** a power switch is turned on. At block **1004,** the inhaler (for example, inhaler **100, 200, 400, 1300**) enters the start state. The flowchart follows from a condition in which the inhaler has already been loaded with a capsule and primed (capsule punctured, flow inlets in position).

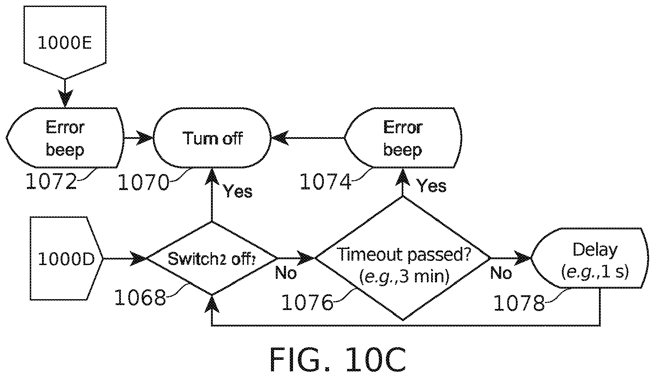

At block **1006,** a determination is made whether or not the inhaler battery needs replacement and/or recharge. If so, a warning indication (such as about 5 seconds of a blinking red LED) is shown at block **1008.** At block **1010,** a determination is made whether or not there is enough battery power remaining to efficiently deliver the current dose. If so, operations continue at block **1012,** with an indication (such as a blue LED) that the system is currently operable. Otherwise, operation continues at flowchart jump location **1000E** (*Figure 10C*)*,* described hereinbelow.

At block **1014,** a timer is started, and polling begins for a minimum flow threshold to be exceeded. At block **1016,** flow sensor data is polled. At block **1018,** a determination is made whether the flow sensor data shows that current flow through the inhaler (due to patient inhalation) exceeds a minimum threshold for activation. If not, operations continue with a delay (for example of 100 msec) at block **1020.**

At block **1022,** a determination is made whether a timeout delay (for example, 10 minutes) has expired. If not, the polling loop continues at block **1016.**

However, if the timeout has occurred, an error indication (for example, a red LED and an error beep) is presented to the user at block **1024.** At block **1026,** use data including, for example, date, time, use number, and a failure code are recorded. Then, at block **1028,** a new timeout timer is started, and operations continue at flowchart jump location **1000D** (*Figure 10C*)*,* described hereinbelow.

Returning to block **1018:** if the minimum flow threshold is exceeded, operations continue at block **1030,** in some embodiments, at which use data including, for example, date, time, use number, and a use number are recorded. Optionally, the flow threshold is set to be a threshold which is expected to be exceeded at some point during a normal breath cycle (that is, an inspiration which does not require special alteration of the patient's breathing pattern).

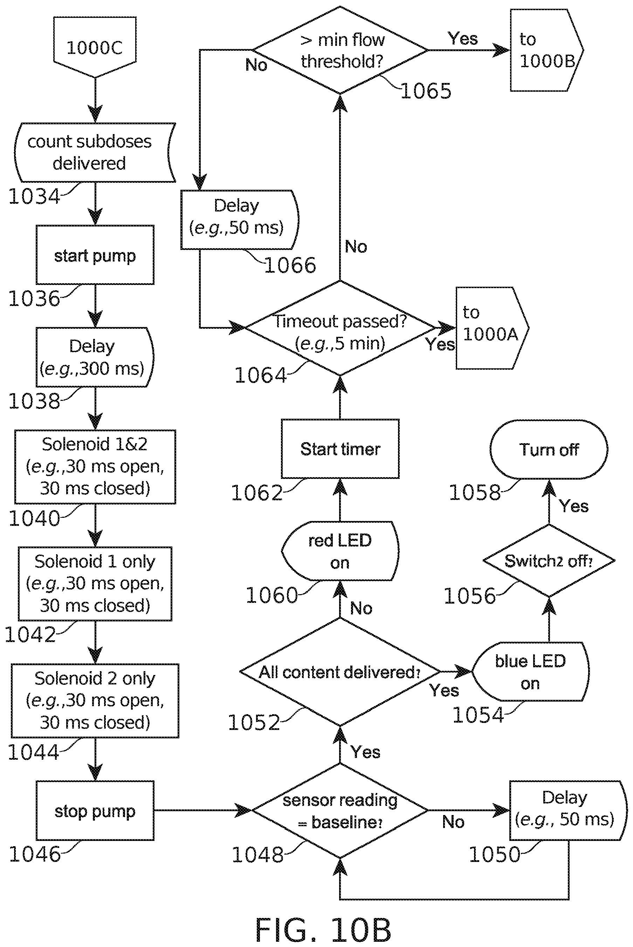

At block **1032,** an indication of activation is shown (for example, illumination of a green LED). The flowchart continues with dose delivery at flowchart jump location **1000C** (*Figure 10B*)*.*

Flowchart jump location **1000C** (*Figure 10B*) leads into block **1034,** at which a count of the number of sub-doses delivered is incremented (and/or a count of sub-doses remaining is decremented). The "sub-doses" are releases of a portion of the medicament contained by a capsule. In some embodiments, administration over several breaths is divided, for example, into **5, 10,** or **20** sub-doses, or a larger, smaller, or intermediate number of sub-doses. This potentially allows drug dispersed into a normal breathing pattern (which does not have an extended inspiration period and/or does not have an inspiration period of dependable length) to be dependably delivered to the patient, rather than exhaled in part. Additionally or alternatively, a potential advantage of splitting drug delivery into sub-doses is to prevent administering enough powder to trigger a cough reflex. This is particularly important for a patient having reduced voluntary control of their respiration.

At block **1036,** a pump is activated, and after a delay at block **1038** (of, for example, 300 msec) to allow accumulation of sufficient pressure and/or arrival at a targeted respiration point, a sequence of solenoid valve operations commences, for delivering compressed air to the dry powder capsule.

The sequence given here is exemplary, assuming two solenoid valves operated in pulses. At block **1040,** both solenoids are opened, and gas vents from the pressure source accumulated from the pump and into each gas delivery lumen which the solenoids gate. Each lumen itself has one or more apertures into the capsule, having been positioned there during inhaler priming. The timing of opening is, for example, 30 msec open, followed by 30 msec closed. It is to be understood that another interval is used in some embodiments, for example, 10 msec, 15 msec, 50 msec, or another greater, lesser or intermediate value. It is also to be understood that the open and closed intervals can be the same or different.

At block **1042,** only the first solenoid is open (and then closed), resulting in a different gas flow pattern inside the capsule. The timing may be the same as for block **1040,** or different. At block **1044,** only the second solenoid is open (and then closed). It is to be understood that the pattern of solenoids being opened is optionally presented in any order, repeating or omitting one of the patterns mentioned, and/or adding a further pattern. Opening and closing of solenoids optionally repeats for a number of cycles in the same or different combinations and/or order of combinations, with the same and/or different periods of opening and/or closing in each cycle. However, as configured for multi-inspiration use, the pattern of release defined by blocks **1038-1044** is generally defined to be brief. Potentially, this ensures that release coincides with natural inhalation by the patient. Each brief release period moreover is presumed to release only a portion of the total dose, and is therefore repeated over a sequence of inspirations, as now described.

At block **1046,** active dispersion (for the current inspiration) is done, and the pump operation is stopped.

At block **1048,** a sensor reading is taken of the inhalation flow rate, and a determination is made whether the inhalation flow rate has returned to baseline. If not, then at block **1050,** a polling delay (for example, 50 msec) is introduced before the sensor baseline determination is made again.

At block **1052,** a determination is made whether all content has been delivered. The determination is made, for example, based on whether or not all scheduled sub-doses have been provided. In some examples, the determination is made by checking the count value adjusted at block 1034 against a target value (for example, 0, if the count is made by decrementing, or a register value if the count is made by incrementing).

If all content is delivered, then at block **1054,** an indication (such as a blue LED) is made, at block **1056,** the inhaler power switch is polled until switch to the off position, and at block **1058** the inhaler is shut down.

If content remains to be delivered, then at block **1060,** an indication (such as a red LED) is provided, and a new timeout timer started at block **1062.**

At block **1064,** if the timeout value (for example, 5 minutes) has passed, the flow chart branches to flowchart jump location **1000A** (*Figure 10A*)*,* which in turn leads to the error indications of block **1024.**

Otherwise, at block **1065,** the determination is again made as to whether or not the minimum respiratory flow threshold has been passed. If not, there is a delay (for example, 50 msec) at block **1066,** and flow returns to the timeout test at block **1064.** If so, then the flowchart branches to flowchart jump location **1000B** (*Figure 10A*)*,* from which the operations of sub-dose dispersal are repeated. Optionally, sub-doses are logged as well as the main does - in this case, the repeat branch returns to block **1030,** to allow re-logging, before the next phase of drug administration.

Remaining to be described is the error-condition shut down routine of *Figure 10C**,* and entered from either jump location **1000D** (near block **1068**) or **1000E** (leading to block **1072**).

At block **1072,** an error indication (such as a beep) is made, and the system turns itself off at block **1070.**

At block **1068,** the position of the user power switch is determined. If not, then the inhaler is turned off at block **1070.**

Otherwise, at block **1076,** a timeout check determines if sufficient time has passed (for example, three minutes) since the error to shut down automatically. If not, then there is a delay at block **1078** (of, for example, one second), and the power switch state test at block **1068** is repeated. If there has been a timeout, then at block **1074** an error indication (such as a beep) is provided to the user, and the system shuts itself down at block **1070.**

### Alternative Single-Inhalation Operation

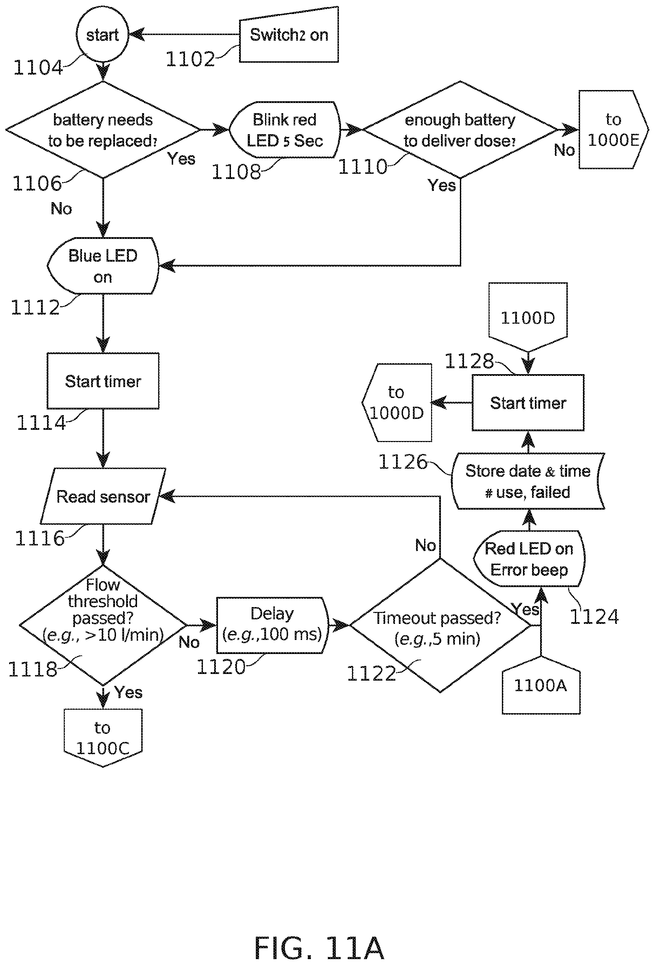

Reference is now made to *Figures 11A-11D**,* which together comprise a schematic illustration of an exemplary method for operating an inhaler with a disposable head for delivering dry powder medicament during a single inhalation of a patient, according to some examples. Typically, but not exclusively, this flow of operations is used with an inhaler operated together with a mouthpiece.

As for *Figures 10A-10C**,* this flow of operations is for use with an inhaler having a disposable head section. A general difference from a fully disposable inhaler design is that the primary limitation on extended use is battery charge, rather than number of uses. *Figures 11A-11D* also provide extended detail relating to timing of valve operation during medicament dispersal, and of other timings related to error conditions, timeouts, and/or polling cycles.

Although the flow of operations is described in connection with specific timings for various operations (for example 30 msec, 100 msec, 5 sec, 10 sec, 5 min, 10 min), it is to be understood that these timings should be taken as exemplary and not limiting. Although the flow of operations is described in connection with specific indications (for example blue LED, green LED, red LED, beep, blink), it is to be understood that these indications should be taken as exemplary and not limiting. Thresholds for activation (described, for example, in relation to a 10 1/minute inspiration rate) should also be understood to be variable to other settings: for example, 1 1/min, 5 1/min, 15 1/min, or another greater, lesser, or intermediate inspiration rate. It should be understood more generally that this flowchart of operations is only one particular example of operations for medicament delivery, and that other flows of operations comprising use of the invention, but having more, fewer, and/or different blocks also represent embodiments of the invention.

The flowchart begins with *Figure 11A**.* Blocks **1102-1116** are generally the same as for corresponding blocks **1002-1016** of *Figure 10A**.* The flowchart follows from a condition in which the inhaler has already been loaded with a capsule and primed (capsule punctured, flow inlets in position).

At block **1118,** a determination is made whether the flow sensor data shows that current flow through the inhaler (due to patient inhalation) exceeds a minimum threshold for activation (for example, 10 1/min). If not, operations continue with a delay (for example of 100 msec) at block **1120.**

Continuing to block **1122,** a determination is made whether a timeout delay (for example, 5 minutes) has expired. If not, the polling loop continues at block **1116**.

Blocks **1124-1128** are similar in description to blocks **1024-1028.** It should be noted that the shutdown sequence jumps to location **1000D** (*Figure 10C*)*,* the same as for the flowchart of *Figures 10A-10C**.*

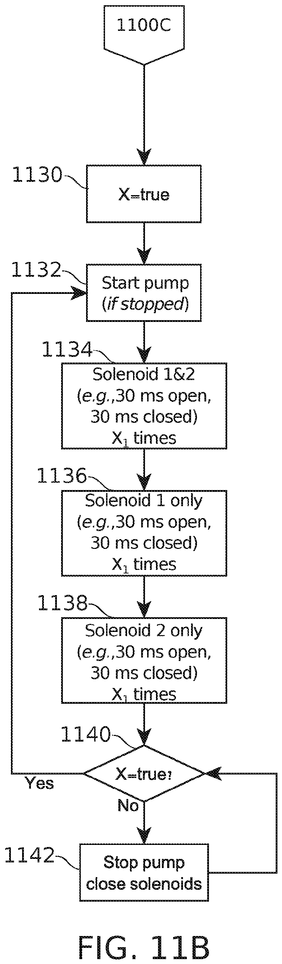

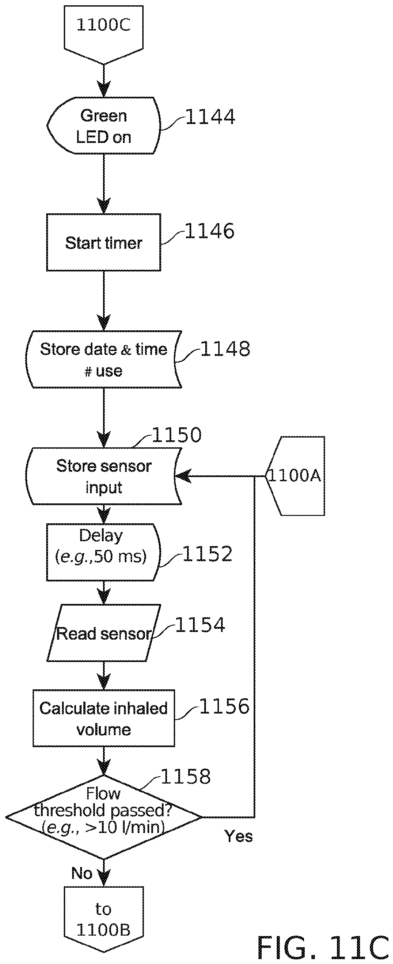

If the breathing threshold has been reached according to the determination at block **1118,** then operations continue from flowchart jump location **1100C** (*Figures 11B* and *11C*). From this point, the flow chart splits into two branches: a medicament dispersal branch at *Figure 11B**,* and a monitoring and recording branch at *Figure 11C**.*

At block **1130,** the medicament dispersal branch begins, and a state variable ("X") is set to indicate that medicament dispersal should continue. This value will be set to false again by a block of the monitoring and recording branch (block **1060**), as described herein below.

At block **1132,** the pump is started. From blocks **1134-1138,** a sequence of solenoid valve operations occurs, for delivering compressed air to the dry powder capsule.

The sequence given here is exemplary, assuming two solenoid valves operated in pulses. At block **1134,** solenoids are opened, and gas vents from the pressure source and into each gas delivery lumen which the solenoids gate. Each lumen itself has one or more apertures into the capsule, having been positioned there during inhaler priming. The timing of opening is, for example, 30 msec open, followed by 30 msec closed. It is to be understood that another interval is used in some embodiments, for example, 10 msec, 15 msec, 50 msec, or another greater, lesser or intermediate value. It is also to be understood that the open and closed intervals can be the same or different. In some embodiments, the opening and closing is repeated by some number of times, for example "X₁" times in total, where X₁ is an integer such as 1, 2, 3, 4, or another number. X₁ is optionally but not necessarily the same for each flow regime.

At block **1136,** only the first solenoid is open (and then closed), resulting in a different gas flow pattern inside the capsule. The timing may be the same as for block **1134,** or different. At block **1138,** only the second solenoid is open (and then closed). The choice of flow regimes and their order as shown is exemplary only and not limiting; other orders, combinations, and/or regimes are also embodiments of the invention.

At block **1140,** a determination is made, based on inspection of state variable "X" as to whether or not the sequence of solenoid operations from block **1134-1138** should repeat. If so, it does. Once "X" is set to false (on the control branch), then execution passes to block **1142,** at which the pump is stopped and the solenoids shut down. However periodic polling at block **1140** optionally continues, and if the state variable "X" is set to be true again, the pump is restarted, and operation resumes. If the delivery was successfully given in a single breath, there is normally no need for resumption, however. The cycling terminates when the inhaler is shut down.

Turning now to *Figure 11C*: at block **1144,** the monitoring and recording branch of the flowchart begins (from the second jump point **1100C**). An indication (such as a green LED) is presented to the user which indicates that medicament administration is underway. It should be understood that the parallel branches of control are shown for purposes of explanation, and are optionally implemented with another organization of operations with respect to their serial/parallel ordering.

At block **1146,** a timeout timer is started, and at block **1148,** use data including, for example, date, time, use number, and a use number are recorded.

At block **1150,** sensor input data is stored. After a delay at block **1152** (for example, a delay of 50 msec), the sensor is read again at block **1154,** and the inhaled volume is calculated at block **1156.** As long as the flow is above a threshold (such as 10 1/min), the cycle returns to block **1150** (during which parallel operations manage gas flow via the solenoids which continue to open and close to release gas into the capsule).

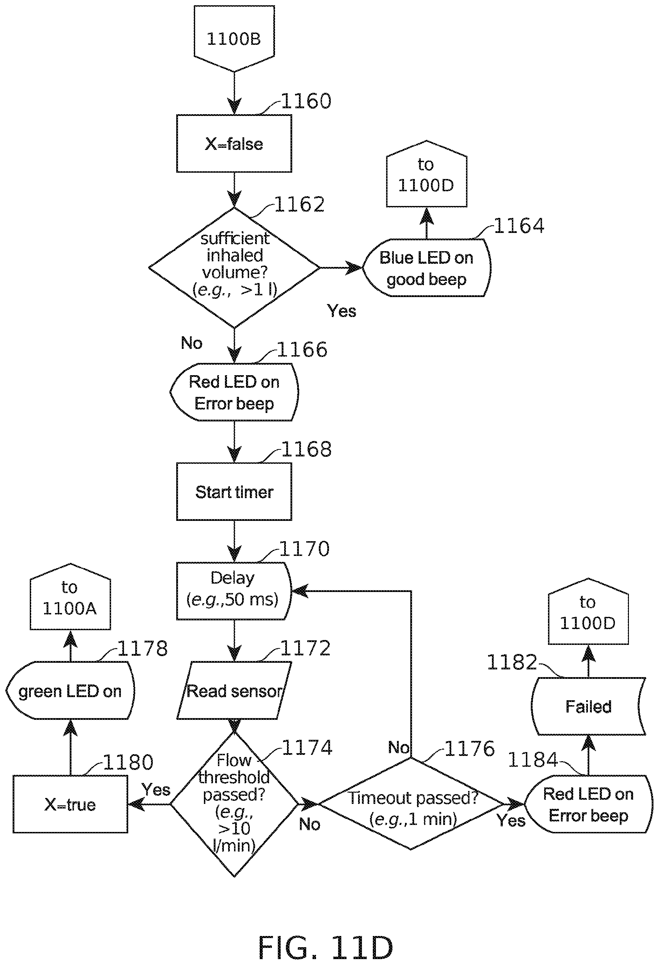

When the flow test at block **1158** shows that flow has fallen below the threshold for inhaler operation, the flowchart operations branch to flowchart jump location **1100B** *(**Figure 11D*)*,* leading to block **1160.**

At block **1160,** the "X" state variable is set false (which eventually leads to at least temporary termination of gas delivery operations to the capsule).

At block **1162,** the data recorded while cycling among blocks **1150-1158** is examined, and it is determined if the inhaled volume is at least some minimum value (for example, 1 liter). If so, then at block **1164,** a success indication (such as a blue LED illumination) is made, and execution continues at flowchart jump location **1100D** (leading to block **1128,** and eventually shut down of the inhaler).

Otherwise, at block **1166,** an indication that inhalation should be repeated to ensure full drug delivery is made (for example, a red LED with a beep). At block **1168,** a new timeout timer is started. After a delay at block **1170** (of, for example, 50 msec), the flow rate sensor is read at block **1172,** and the determination made at block **1174** as to whether the minimum flow rate threshold has been again exceeded. This branch of operations is normally entered only in the event of an exception - the usual operation of the device should lead to the full amount of volume being inspired during the first continuous breath exceeding the starting flow threshold.

As long as the flow threshold is not exceeded, then at block **1176,** a timeout check determines if more than some period has passed since the timeout timer was set (for example, more than one minute). If so, an error condition has occurred (drug not fully delivered). At block **1184,** an error indication is made (for example, a red LED illumination and beep); a failed state is set at block **1182,** and the shutdown sequence is entered through flowchart jump location **1100D.**

Otherwise, at block **1180,** the state variable "X" is again set to true. At block **1178,** the inhaler active indication is set (for example, a green LED illumination) and control of the flowchart returns to flowchart jump location **1100A** of *Figure 11B**,* leading into block **1150.**

As used herein with reference to quantity or value, the term "about" means "within ±10% of".

The terms "comprises", "comprising", "includes", "including", "having" and their conjugates mean: "including but not limited to".

The term "consisting of" means: "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

The words "example" and "exemplary" are used herein to mean "serving as an example, instance or illustration". Any embodiment described as an "example" or "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features except insofar as such features conflict.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", *etc*.; as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

## Claims

1. An inhaler (400) for delivering dry powder medicament contained in a capsule (420), the inhaler (400) comprising:
a capsule chamber (404) sized to fittingly hold said capsule (420) within a capsule-holding volume (404);
at least one needle (402) within the inhaler (400), the needle comprising a gas lumen having at least one gas outlet (304); and
an actuating mechanism configured to move with respect to one another the capsule chamber (404) and the at least one needle (402);
wherein the actuating mechanism is actuatable to prime the inhaler (400) by movement of the needle (402) with respect to the capsule chamber (404) along a path to introduce a portion of the needle (402) across a first boundary location of the capsule-holding volume (404), and into an interior of the capsule-holding volume (404); and
wherein the actuating mechanism continues actuated movement of the needle (402) along the path with respect to the capsule chamber (404) to move said portion of the needle (402) from said interior to a second boundary location of the capsule-holding volume (404), and then to place said portion of the needle (402) to extend past the second boundary location to exit the capsule-holding volume (404);
**characterized in that**:
further continued actuation of the actuating mechanism retracts the portion of the needle (402) including the at least one gas outlet (304), back into the interior and away from the second boundary location; and
wherein the inhaler (400) is configured to disperse the dry powder medicament from an exit aperture (310) left open by the retracted needle in the capsule (420) wall at the second boundary location, while the portion of the needle (402) including the at least one gas outlet (304) remains retracted back into the interior, and when the inhaler (400) is primed with the capsule (420) placed within the capsule-holding volume (404).

2. The inhaler (400) of claim 1, wherein: when the capsule (420) is placed within the capsule-holding volume (404), the actuated movement of the needle along the path to extend past the second boundary location to exit the capsule holding volume (404) forces the portion of the needle (402) against a wall of the capsule (420) from within said interior to form a capsule outlet (310) for the dry powder medicament.

3. The inhaler (400) of claim 2, wherein a cross-section of the portion of the needle (402) movable by actuated movement to extend past the second boundary location defines the size of the capsule outlet (310).

4. The inhaler (400) of claim 3, wherein the portion of the needle (402) moveable by actuated movement to extend past the second boundary location comprises cross-sections of at least two different diameters on a respective at least two longitudinal sections, each said longitudinal section having a different constant-size diameter along its longitudinal extent.

5. The inhaler (400) of claim 3, wherein each needle (402) cross-section configured to cross the second boundary location comprises a diameter at least as large as any diameter of a more distal portion of the needle (402).

6. The inhaler (400) of any one of claims 1-5, wherein the inhaler (400) comprises a compressed gas source (116, 118) and a delivery system (120) for gas from said compressed gas source (116, 118) to the lumen of the needle (402).

7. The inhaler (400) of claim 6, comprising at least two spatially separated said gas outlets (304) on the at least one needle (402).

8. The inhaler (400) of claim 7, comprising at least two of the needles (402), and wherein the spatially separated gas outlets (304) are distributed on the at least two different needles (402) and positioned within a region of said capsule-holding volume (404) sized to hold a single capsule (420).

9. The inhaler (400) of claim 7, comprising at least one valve for separately controlling gas delivery via each of said gas outlets (304).

10. The inhaler (400) of claim 6, wherein the actuating mechanism is actuatable after a first delivery of the gas from the compressed gas source (116, 118) to position said gas outlet (304) in at least an additional position within the capsule-holding volume (404) from which gas is delivered into the capsule-holding volume (404), when the capsule (420) is positioned in the capsule-holding volume (404), and while the capsule (420) remains so-positioned.

11. The inhaler (400) of any one of claims 6-10, further comprising a controller (108) functionally connected with said gas delivery system (120) for controlling delivery of compressed gas to said gas outlet (304).

12. A detachable module (1302) fittable to an inhaler (1300) for delivering dry powder medicament contained in a capsule (210), the module (1302) comprising:
a capsule chamber (1311) sized to fittingly hold said capsule (210) within a capsule-holding volume (124);
at least one needle (122) within the detachable module (1302) comprising a gas lumen having at least one gas outlet (304); and
an actuating mechanism configured to move with respect to one another the capsule chamber (1311) and the at least one needle (122);
wherein the actuating mechanism is actuatable to prime the inhaler (1300) by movement of the needle (122) with respect to the capsule chamber (1311) along a path to introduce a portion of the needle (122) across a first boundary location of the capsule-holding volume (1311), and into an interior of the capsule-holding volume (1311); and
wherein the actuating mechanism continues actuated movement of the needle (122) along the path with respect to the capsule chamber (1311) to move said portion of the needle (122) from said interior to a second boundary location of the capsule-holding volume (1311), and then to place said portion of the needle (402) to extend past the second boundary location to exit the capsule-holding volume (404);
**characterized in that**:
further continued actuation of the actuating mechanism retracts the portion of the needle (122) including the at least one gas outlet (304), back into the interior and away from the second boundary location; and
wherein the inhaler (1300) is configured to disperse the dry powder medicament from an exit aperture (310) left open by the retracted needle in the capsule (420) wall at the second boundary location, while the portion of the needle (122) including the at least one gas outlet (304) remains retracted back into the interior, and when the inhaler (1300) is primed with the capsule (210) placed within the capsule-holding volume (1311).

13. A kit comprising the module (1302) of claim 12, and at least ten capsules (210) containing dry powder medicament.

14. A method of preparing an inhaler capsule (210) containing dry powder medicament with a capsule outlet (310) for the medicament, the method comprising:
introducing a needle (122) comprising a gas lumen having at least one gas outlet (304) into an interior of the capsule (210); and
puncturing a wall of the inhaler capsule (210) by forcing the needle (122) against the wall from the capsule interior to define the capsule outlet (310);
**characterized by**:
withdrawing the needle (122) to de-obstruct the capsule outlet (310), positioning the at least one gas outlet (304) within the capsule interior; and
directing gas into the at least one gas outlet (304) while it remains in the interior and the capsule outlet (310) is de-obstructed, thereby releasing the dry powder medicament from the capsule (210).

15. The method of claim 14, wherein no more than 20% of the wall of the capsule (210) displaced to form the capsule outlet (310) is within the capsule interior after formation of the capsule outlet (310).

## Patentansprüche

1. Inhalator (400) zur Abgabe eines Trockenpulvermedikaments, das in einer Kapsel (420) enthalten ist, wobei der Inhalator (400) umfasst:
eine Kapselkammer (404), die so bemessen ist, dass sie die Kapsel (420) in einem Kapselhaltevolumen (404) passend festhält;
mindestens eine Nadel (402) innerhalb des Inhalators (400), wobei die Nadel ein Gaslumen umfasst, das mindestens einen Gasauslass (304) aufweist; und
einen Betätigungsmechanismus, der konfiguriert ist, die Kapselkammer (404) und die mindestens eine Nadel (402) im Bezug zueinander zu bewegen;
wobei der Betätigungsmechanismus auslösbar ist, um den Inhalator (400) durch die Bewegung der Nadel (402) in Bezug auf die Kapselkammer (404) entlang eines Weges vorzubereiten, um ein Teilstück der Nadel (402) über eine erste Begrenzungsposition des Kapselhaltevolumens (404) hinweg und in das Innere des Kapselhaltevolumens (404) einzuführen; und
wobei der Betätigungsmechanismus mit der ausgelösten Bewegung der Nadel (402) entlang des Pfades in Bezug auf die Kapselkammer (404) fortfährt, um das Teilstück der Nadel (402) von dem Innenraum zu einer zweiten Begrenzungsposition des Kapselhaltevolumens (404) zu bewegen, und anschließend das Teilstück der Nadel (402) so anzuordnen, sodass es sich über die zweite Begrenzungsposition hinaus erstreckt, um aus dem Kapselhaltevolumen (404) auszutreten;
**dadurch gekennzeichnet, dass**:
eine weitere fortgeführte Betätigung des Betätigungsmechanismus das Teilstück der Nadel (402), welches den mindestens einen Gasauslass (304) umfasst, zurück in den Innenraum und weg von der zweiten Begrenzungsposition zieht; und
wobei der Inhalator (400) konfiguriert ist, das Trockenpulvermedikament zu dispergieren, von einer Austrittsöffnung (310), die durch die zurückgezogene Nadel in der Wand der Kapsel (420) an der zweiten Begrenzungsposition offen gelassen wird, während das Teilstück der Nadel (402), welches den mindestens einen Gasauslass (304) umfasst, zurückgezogen in dem Innenraum verbleibt, und wenn der Inhalator (400) mit der Kapsel (420), die innerhalb des Kapselhaltevolumens (404) angeordnet ist, vorbereitet wird.

2. Inhalator (400) nach Anspruch 1, wobei: wenn die Kapsel (420) innerhalb des Kapselhaltevolumens (404) angeordnet ist, die ausgelöste Bewegung der Nadel entlang des Pfades, um sich über die zweite Begrenzungsposition hinaus zu erstrecken, um aus dem Kapselhaltevolumen (404) auszutreten, das Teilstück der Nadel (402) von dem Inneren gegen eine Wand der Kapsel (420) gedrückt, um einen Kapselauslass (310) für das Trockenpulvermedikament auszubilden.

3. Inhalator (400) nach Anspruch 2, wobei ein Querschnitt des Teilstücks der Nadel (402), das durch eine ausgelöste Bewegung bewegbar ist, um sich über die zweite Begrenzungsposition hinaus zu erstrecken, die Größe des Kapselauslasses (310) definiert.

4. Inhalator (400) nach Anspruch 3, wobei das Teilstück der Nadel (402), das durch eine ausgelöste Bewegung bewegbar ist, um sich über die zweite Begrenzungsposition hinaus zu erstrecken, Querschnitte von mindestens zwei unterschiedlichen Durchmessern auf jeweils mindestens zwei Längsabschnitten umfasst, wobei jeder Längsabschnitt einen unterschiedlichen, konstant-großen Durchmesser entlang seiner Längserstreckung aufweist.

5. Inhalator (400) nach Anspruch 3, wobei jeder Querschnitt der Nadel (402), der konfiguriert ist, die zweite Begrenzungsposition zu durchqueren, einen Durchmesser umfasst, der mindestens so groß ist wie jeder Durchmesser eines weiter distal gelegenen Teilstücks der Nadel (402).

6. Inhalator (400) nach einem der Ansprüche 1 bis 5, wobei der Inhalator (400) eine Druckgasquelle (116, 118) und ein Abgabesystem (120) für Gas von der Druckgasquelle (116, 118) zu dem Lumen der Nadel (402) umfasst.

7. Inhalator (400) nach Anspruch 6, der mindestens zwei räumlich getrennte Gasauslässe (304) an der mindestens einen Nadel (402) umfasst.

8. Inhalator (400) nach Anspruch 7, der mindestens zwei der Nadeln (402) umfasst, und wobei die räumlich getrennten Gasauslässe (304) auf den mindestens zwei verschiedenen Nadeln (402) verteilt sind und innerhalb eines Bereichs des Kapselhaltevolumens (404) positioniert sind, das dimensioniert ist, eine einzelne Kapsel (420) festzuhalten.

9. Inhalator (400) nach Anspruch 7, der mindestens ein Ventil zum getrennten Steuern der Gasabgabe über jeden der Gasauslässe (304) umfasst.

10. Inhalator (400) nach Anspruch 6, wobei der Betätigungsmechanismus nach einer ersten Abgabe des Gases von der Druckgasquelle (116, 118) betätigbar ist, um den Gasauslass (304) in mindestens einer zusätzlichen Position innerhalb des Kapselhaltevolumens (404) zu positionieren, von der Gas in das Kapselhaltevolumen (404) abgegeben wird, wenn die Kapsel (420) in dem Kapselhaltevolumen (404) positioniert ist und während die Kapsel (420) so positioniert verbleibt.

11. Inhalator (400) nach einem der Ansprüche 6 bis 10, der ferner eine Steuereinheit (108), die funktionell mit dem Gasabgabesystem (120) verbunden ist, um die Abgabe von komprimiertem Gas zu dem Gasauslass (304) zu steuern, umfasst.

12. Abtrennbares Modul (1302), das an einen Inhalator (1300) anpassbar ist, um ein Trockenpulvermedikament abzugeben, das in einer Kapsel (210) enthalten ist, wobei das Modul (1302) umfasst:
eine Kapselkammer (1311), die so bemessen ist, dass sie die Kapsel (210) in einem Kapselhaltevolumen (124) passend festhält;
mindestens eine Nadel (122) innerhalb des abtrennbaren Moduls (1302), wobei die Nadel ein Gaslumen aufweist, das mindestens einen Gasauslass (304) aufweist; und
einen Betätigungsmechanismus, der konfiguriert ist, die Kapselkammer (1311) und die mindestens eine Nadel (122) im Bezug zueinander zu bewegen;
wobei der Betätigungsmechanismus auslösbar ist, um den Inhalator (1300) durch die Bewegung der Nadel (122) in Bezug auf die Kapselkammer (1311) entlang eines Weges vorzubereiten, um ein Teilstück der Nadel (122) über eine erste Begrenzungsposition des Kapselhaltevolumens (1311) hinweg und in das Innere des Kapselhaltevolumens (1311) einzuführen; und
wobei der Betätigungsmechanismus mit der ausgelösten Bewegung der Nadel (122) entlang des Pfades in Bezug auf die Kapselkammer (1311) fortfährt, um das Teilstück der Nadel (122) von dem Innenraum zu einer zweiten Begrenzungsposition des Kapselhaltevolumens (1311) zu bewegen, und anschließend das Teilstück der Nadel (402) so anzuordnen, sodass es sich über die zweite Begrenzungsposition hinaus erstreckt, um aus dem Kapselhaltevolumen (404) auszutreten;
**dadurch gekennzeichnet, dass**:
eine weitere fortgeführte Betätigung des Betätigungsmechanismus das Teilstück der Nadel (122), welches den mindestens einen Gasauslass (304) umfasst, zurück in den Innenraum und weg von der zweiten Begrenzungsposition zieht; und
wobei der Inhalator (1300) konfiguriert ist, das Trockenpulvermedikament zu dispergieren, von einer Austrittsöffnung (310), die durch die zurückgezogene Nadel in der Wand der Kapsel (420) an der zweiten Begrenzungsposition offen gelassen wird, während das Teilstück der Nadel (122), welches den mindestens einen Gasauslass (304) umfasst, zurückgezogen in dem Innenraum verbleibt, und wenn der Inhalator (1300) mit der Kapsel (210), die innerhalb des Kapselhaltevolumens (1311) angeordnet ist, vorbereitet wird.

13. Kit, umfassend das Modul (1302) nach Anspruch 12 und mindestens zehn Kapseln (210), die ein Trockenpulvermedikament enthalten.

14. Verfahren zur Herstellung einer Inhalatorkapsel (210), die ein Trockenpulvermedikament enthält, mit einem Kapselauslass (310) für das Medikament, wobei das Verfahren umfasst:
Einführen einer Nadel (122), die ein Gaslumen umfasst, das mindestens einen Gasauslass (304) aufweist, in ein Inneres der Kapsel (210); und
Punktieren einer Wand der Inhalatorkapsel (210) durch Drücken der Nadel (122) gegen die Wand von dem Kapselinneren, um den Kapselauslass (310) zu definieren;
**gekennzeichnet durch**:
Zurückziehen der Nadel (122), um den Kapselauslass (310) zu entsperren, Positionieren des mindestens einen Gasauslasses (304) innerhalb des Kapselinneren; und
Leiten von Gas in den mindestens einen Gasauslass (304), während er im Inneren verbleibt und der Kapselauslass (310) entsperrt ist, wodurch das Trockenpulvermedikament aus der Kapsel (210) freigesetzt wird.

15. Verfahren nach Anspruch 14, wobei nicht mehr als 20 % der Wand der Kapsel (210), die verlagert wird, um den Kapselauslass (310) auszubilden, nach der Ausbildung des Kapselauslasses (310) innerhalb des Kapselinneren ist.

## Revendications

1. Inhalateur (400) pour délivrer un médicament en poudre sèche contenu dans une capsule (420), l'inhalateur (400) comprenant :
une chambre de capsule (404) dimensionnée pour former une complémentarité de formes avec la forme de ladite capsule (420) au sein d'un volume de support de capsule (404) ;
au moins une aiguille (402) au sein de l'inhalateur (400), l'aiguille comprenant une lumière de gaz ayant au moins une sortie de gaz (304) ; et
un mécanisme d'actionnement configuré pour déplacer par rapport l'un à l'autre la chambre de capsule (404) et l'au moins une aiguille (402) ;
dans lequel le mécanisme d'actionnement est actionnable pour amorcer l'inhalateur (400) par mouvement de l'aiguille (402) par rapport à la chambre de capsule (404) le long d'une voie pour introduire une partie de l'aiguille (402) à travers un premier emplacement limite du volume de support de capsule (404), et dans un intérieur du volume de support de capsule (404) ; et
dans lequel le mécanisme d'actionnement continue le mouvement actionné de l'aiguille (402) le long de la voie par rapport à la chambre de capsule (404) pour déplacer ladite partie de l'aiguille (402) dudit intérieur vers un second emplacement limite du volume de support de capsule (404), et puis pour placer ladite partie de l'aiguille (402) pour s'étendre après le second emplacement limite pour sortir le volume de support de capsule (404) ;
**caractérisé en ce que** :
l'actionnement continu ultérieur du mécanisme d'actionnement rétracte la partie de l'aiguille (402) incluant l'au moins une sortie de gaz (304), dans l'intérieur et à l'écart du second emplacement limite ; et
dans lequel l'inhalateur (400) est configuré pour disperser le médicament en poudre sèche à partir d'une ouverture de sortie (310) laissée ouverte par l'aiguille rétractée dans la paroi de la capsule (420) au niveau du second emplacement limite, tandis que la partie de l'aiguille (402) incluant l'au moins une sortie de gaz (304) reste rétractée dans l'intérieur, et lorsque l'inhalateur (400) est amorcé avec la capsule (420) placée au sein du volume de support de capsule (404).

2. Inhalateur (400) selon la revendication 1, dans lequel : lorsque la capsule (420) est placée au sein du volume de support de capsule (404), le mouvement actionné de l'aiguille le long de la voie pour s'étendre après le second emplacement limite pour sortir le volume de support de capsule (404) force la partie de l'aiguille (402) contre une paroi de la capsule (420) à partir du sein dudit intérieur pour former une sortie de capsule (310) pour le médicament en poudre sèche.

3. Inhalateur (400) selon la revendication 2, dans lequel une section transversale de la partie de l'aiguille (402) déplaçable par mouvement actionné pour s'étendre après le second emplacement limite définit la dimension de la sortie de capsule (310).

4. Inhalateur (400) selon la revendication 3, dans lequel la partie de l'aiguille (402) déplaçable par mouvement actionné pour s'étendre après le second emplacement limite comprend des sections transversales d'au moins deux diamètres différents respectivement sur au moins deux sections longitudinales, chaque dite section longitudinale ayant un diamètre de dimension constante différent le long de son étendue longitudinale.

5. Inhalateur (400) selon la revendication 3, dans lequel chaque section transversale d'aiguille (402) configurée pour traverser le second emplacement limite comprend un diamètre au moins aussi important que tout diamètre d'une partie plus distale de l'aiguille (402).

6. Inhalateur (400) selon l'une quelconque des revendications 1 à 5, dans lequel l'inhalateur (400) comprend une source de gaz comprimé (116, 118) et un système de délivrance (120) pour le gaz à partir de ladite source de gaz comprimé (116, 118) vers la lumière de l'aiguille (402).

7. Inhalateur (400) selon la revendication 6, comprenant au moins deux desdites sorties de gaz (304) séparées dans l'espace sur l'au moins une aiguille (402).

8. Inhalateur (400) selon la revendication 7, comprenant au moins deux des aiguilles (402), et dans lequel les sorties de gaz (304) séparées dans l'espace sont réparties sur les au moins deux aiguilles (402) différentes et positionnées au sein d'une région dudit volume de support de capsule (404) dimensionné pour supporter une capsule (420) unique.

9. Inhalateur (400) selon la revendication 7, comprenant au moins une soupape pour contrôler séparément la délivrance de gaz via chacune desdites sorties de gaz (304).

10. Inhalateur (400) selon la revendication 6, dans lequel le mécanisme d'actionnement est actionnable après une première délivrance du gaz à partir de la source de gaz comprimé (116, 118) pour positionner ladite sortie de gaz (304) dans au moins une position additionnelle au sein du volume de support de capsule (404) à partir de laquelle le gaz est délivré dans le volume de support de capsule (404), et tandis que la capsule (420) reste ainsi positionnée.

11. Inhalateur (400) selon l'une quelconque des revendications 6 à 10, comprenant en outre un contrôleur (108) connecté de manière fonctionnelle audit système de délivrance de gaz (120) pour commander la délivrance de gaz comprimé vers ladite sortie de gaz (304).

12. Module détachable (1302) adaptable sur un inhalateur (1300) pour délivrer un médicament en poudre sèche contenu dans une capsule (210), le module (1302) comprenant :
une chambre de capsule (1311) dimensionnée pour former une complémentarité de formes avec la forme de ladite capsule (210) au sein d'un volume de support de capsule (124) ;
au moins une aiguille (122) au sein du module détachable (1302) comprenant une lumière de gaz ayant au moins une sortie de gaz (304) ; et
un mécanisme d'actionnement configuré pour déplacer par rapport l'un à l'autre la chambre de capsule (1311) et l'au moins une aiguille (122) ;
dans lequel le mécanisme d'actionnement est actionnable pour amorcer l'inhalateur (1300) par mouvement de l'aiguille (122) par rapport à la chambre de capsule (1311) le long d'une voie pour introduire une partie de l'aiguille (122) à travers un premier emplacement limite du volume de support de capsule (1311), et à l'intérieur d'un intérieur du volume de support de capsule (1311) ; et
dans lequel le mécanisme d'actionnement continue le mouvement actionné de l'aiguille (122) le long de la voie par rapport à la chambre de capsule (1311) pour déplacer ladite partie de l'aiguille (122) dudit intérieur vers un second emplacement limite du volume de support de capsule (1311), et puis pour placer ladite partie de l'aiguille (402) pour s'étendre après le second emplacement limite pour sortir le volume de support de capsule (404) ;
**caractérisé en ce que** :
l'actionnement continu ultérieur du mécanisme d'actionnement rétracte la partie de l'aiguille (122) incluant l'au moins une sortie de gaz (304), dans l'intérieur et à l'écart du second emplacement limite ; et
dans lequel l'inhalateur (1300) est configuré pour disperser le médicament en poudre sèche à partir d'une ouverture de sortie (310) laissée ouverte par l'aiguille rétractée dans la paroi de la capsule (420) au niveau du second emplacement limite, tandis que la partie de l'aiguille (122) incluant l'au moins une sortie de gaz (304) reste rétractée dans l'intérieur, et lorsque l'inhalateur (1300) est amorcé avec la capsule (210) placée au sein du volume de support de capsule (1311).

13. Kit comprenant le module (1302) selon la revendication 12, et au moins dix capsules (210) contenant un médicament en poudre sèche.

14. Procédé de préparation d'une capsule (210) d'inhalateur contenant un médicament en poudre sèche avec une sortie de capsule (310) pour le médicament, le procédé comprenant :
l'introduction d'une aiguille (122) comprenant une lumière de gaz ayant au moins une sortie de gaz (304) au sein d'un intérieur de la capsule (210) ; et
le percement d'une paroi de la capsule (210) d'inhalateur en forçant l'aiguille (122) contre la paroi à partir de l'intérieur de la capsule pour définir la sortie de capsule (310) ;
**caractérisé par** :
le retrait de l'aiguille (122) pour désobstruer la sortie de capsule (310),
le positionnement de l'au moins une sortie de gaz (304) au sein de l'intérieur de la capsule ; et
la direction du gaz dans au moins une sortie de gaz (304) tandis qu'il reste dans l'intérieur et que la sortie de capsule (310) est désobstruée, libérant ainsi le médicament en poudre sèche à partir de la capsule (210).

15. Procédé selon la revendication 14, dans lequel 20 % au plus de la paroi de la capsule (210) déplacée pour former la sortie de capsule (310) est au sein de l'intérieur de la capsule après formation de la sortie de capsule (310).
